# EUROPÄISCHE PATENTSCHRIFT

(11) **EP 4 488 948 B1**
(45) Veröffentlichungstag und Bekanntmachung des Hinweises auf die Patenterteilung: **21.10.2026**
(21) Anmeldenummer: 23183575.2
(22) Anmeldetag: 05.07.2023
(51) Int. Cl.: G06T 12/30, G06T 7/11, G06T 7/194

(54) **VERFAHREN ZUM EINSTELLEN DER SICHTBARKEIT VON OBJEKTEN IN EINEM DURCH STRAHLUNG ERZEUGTEN PROJEKTIONSBILD**
METHOD FOR ADJUSTING THE VISIBILITY OF OBJECTS IN A PROJECTION IMAGE PRODUCED BY RADIATION
PROCÉDÉ DE RÉGLAGE DE LA VISIBILITÉ D'OBJETS DANS UNE IMAGE DE PROJECTION GÉNÉRÉE PAR RAYONNEMENT

(43) Veröffentlichungstag der Anmeldung: 08.01.2025
(73) Patentinhaber: Ziehm Imaging GmbH, 90471 Nürnberg (DE)
(72) Erfinder: Vöth, Tim, 90459 Nürnberg (DE); König, Thomas, 90478 Nürnberg (DE); Hörndler, Klaus, 90482 Nürnberg (DE); Knaupp, Michael, 91244 Reichenschwand (DE); Kachelrieß, Marc, 90419 Nürnberg (DE)
(74) Vertreter: Wittmann, Günther

(56) Entgegenhaltungen:
- US-A1- 2020 184 639
- US-A1- 2023 097 849

## Beschreibung

### Technisches Gebiet

Die Erfindung betrifft eine Vorrichtung und ein Verfahren zur semantischen Kontrastanpassung von Röntgenbildern.

### Hintergrund der Erfindung

Röntgenbilder basieren auf der Abschwächung von Röntgenstrahlung in Materie und können dabei einen sehr hohen Dynamikumfang aufweisen, also einen großen Unterschied zwischen minimalen und maximalen Röntgen- und damit Bildintensitäten. Bei nativer, d.h. unveränderter Darstellung auf einem Anzeigegerät kann es dabei häufig vorkommen, dass die relevanten Bildbereiche nur schwer erkennbar sind oder der lokale Kontrast zu niedrig ist für die Differenzierung von Strukturen. Ein Beispiel ist die Navigation von Instrumenten, beispielsweise Führungsdrähten oder Kathetern, in Blutgefäßen, welche beispielsweise projektiv vor oder hinter der Wirbelsäule liegen und damit im Röntgenbild von dieser überlagert werden.

Es ist bekannt, dass in manchen Fällen die globale Skalierung der Bildintensitäten (insbesondere Grauwerten) hilfreich ist, beispielsweise durch Anpassung von Helligkeit oder Kontrast, oder die Verwendung von Tonwertkurven oder Gammakorrekturen. Diese Verfahren haben den Nachteil, dass sie durch die Anwendung einer global gültigen Abbildungsvorschrift zwischen Eingangs- und Ausgangswerten das gesamte Bild beeinflussen. Eine Möglichkeit zur Kompression der Bilddynamik durch Anhebung der lokalen Kontraste besteht in der Anwendung von Frequenz- bzw. Bandpassfiltern im Bild- oder Fourierraum.

Solche Filter können auch aus einer Kombination multipler Bandpässe bestehen, sie entfernen in der Regel jedoch vor allem niedrige Frequenzen aus dem Fourierraum des Bildes. Da sie hohe Frequenzen damit weitgehend intakt lassen oder möglicherweise sogar anheben, führt dies zu einer Abschwächung der Bildeinflüsse von großen Strukturen, jedoch tendenziell auch zu einer Verstärkung von Bildrauschen relativ zum verbleibenden Bildinhalt. In der Folge kann beispielsweise ein Führungsdraht vor einer Wirbelsäule besser oder überhaupt erst erkannt werden. Im Resultat führen solche Verfahren durch Reduktion des Dynamikumfangs also zu einer besseren Erkennbarkeit mancher Strukturen für einen menschlichen Beobachter, obwohl sie jedoch tatsächlich Bildinformationen entfernen (hier bevorzugt niedrige Frequenzen). Sie haben damit zwar eine Auswirkung auf lokale Kontraste, wirken jedoch ebenfalls global, d.h. überall gleich (von Randeffekten einmal abgesehen). Solcherlei Verfahren sind außerdem aus der Fotografie bekannt, wo sie bei High Dynamic Range (HDR) Aufnahmen als sog. Tone Mapping zum Einsatz kommen, um den hohen Dynamikumfang eines Bildes auf den von Monitoren abbildbaren Bereich zu reduzieren/komprimieren.

### Stand der Technik

Die WO 2019/141544 A1 betrifft ein System zur Bildzerlegung eines anatomischen Projektionsbildes. Das System umfasst ein Datenverarbeitungssystem, das einen Algorithmus zur Zerlegung eines Projektionsbildes implementiert, das durch Bestrahlung eines Teils eines Subjekts mit bildgebender Strahlung erzeugt wird. Ein Körperbereich innerhalb des bestrahlten Teils ist eine dreidimensionale Abschwächungsstruktur einer Abschwächung der bildgebenden Strahlung, wobei die Abschwächungsstruktur ein Mitglied einer vordefinierten Klasse von Abschwächungsstrukturen des Zerlegungsalgorithmus darstellt, wodurch eine Klassifizierung des Körperbereichs dargestellt wird. Das Datenverarbeitungssystem zerlegt die Projektionsbilddaten unter Verwendung der Klassifizierung der Abschwächungsstruktur. Die Dekomposition der Projektionsbilddaten trennt im Wesentlichen den Beitrag des klassifizierten Körperbereichs zum Projektionsbild von dem Beitrag eines weiteren Körperbereichs zu dem Projektionsbild. Der weitere Körperbereich überschneidet sich mit dem klassifizierten Körperbereich im Projektionsbild.

Die WO 2015/197738 A1 betrifft die Sichtbarmachung von zuvor unterdrückten Bildstrukturen in einem Röntgenbild. Ein grafischer Indikator wird auf dem Röntgenbild eingeblendet, um die unterdrückte Bildstruktur anzuzeigen. Die Geräte ermöglichen es, den grafischen Indikator ein- und auszublenden oder zwischen verschiedenen grafischen Darstellungen umzuschalten.

Die US 9,990,743 B2 offenbart, dass Bildverarbeitungstechniken eine Methodik zur Normalisierung medizinischer Bild- und/oder Voxeldaten umfassen können, die unter verschiedenen Aufnahmeprotokollen erfasst wurden, sowie eine Methodik zur Unterdrückung ausgewählter anatomischer Strukturen aus medizinischen Bild- und/oder Voxeldaten, was zu einer verbesserten Erfassung und/oder verbesserten Darstellung anderer anatomischer Strukturen führen kann. Die hier vorgestellte Technologie kann beispielsweise zur verbesserten Erfassung von Knötchen in Computertomographien (CT) eingesetzt werden.

Die US 8,204,292 B2 offenbart, dass ein Verfahren zur Gewinnung einer oder mehrerer Komponenten aus einem Bild das Normalisieren und Vorverarbeiten des Bildes umfassen kann, um ein verarbeitetes Bild zu erhalten. Aus dem verarbeiteten Bild können Merkmale extrahiert werden. Eine auf neuronalen Netzen basierende Regression kann dann auf dem Satz der extrahierten Merkmale durchgeführt werden, um die eine oder mehrere Komponenten vorherzusagen. Diese Techniken lassen sich beispielsweise auf die Extraktion und die Entfernung von Knochenkomponenten aus Röntgenbildern anwenden.

Die US 2023/0097849 A1 offenbart, dass bei einem Verfahren zur Erstellung eines trainierten Modells ein rekonstruiertes Bild erzeugt wird, das durch Rekonstruktion dreidimensionaler Röntgenbilddaten erhalten wird. Ein Projektionsbild wird aus einem dreidimensionalen Modell eines Bildelements durch eine Simulation erzeugt. Das Projektionsbild wird mit dem rekonstruierten Bild überlagert, um ein Überlagerungsbild zu erzeugen. Ein trainiertes Modell wird durch maschinelles Lernen unter Verwendung des überlagerten Bildes und des rekonstruierten Bildes oder des Projektionsbildes erstellt.

### Allgemeine Beschreibung der Erfindung

Die Erfindung stellt sich zur Aufgabe, ein Verfahren und eine Vorrichtung zum Einstellen der Sichtbarkeit von Objekten in einem durch Strahlung erzeugten Projektionsbild zu schaffen.

Die Aufgabe der Erfindung wird durch ein Verfahren nach Anspruch 1 und eine Vorrichtung nach Anspruch 15 gelöst. Die abhängigen Ansprüche beanspruchen Ausführungsformen der Erfindung.

Die vorliegende Erfindung offenbart ein Verfahren zum Einstellen der Sichtbarkeit von Objekten in einem durch Strahlung erzeugten Projektionsbild eines anatomischen Bereichs, bei dem Bilddaten, die das durch Strahlung erzeugte Projektionsbild repräsentieren, in einen Speicher eines Computers oder einer Recheneinheit geladen werden. Ein erstes Teilbild wird aus den Bilddaten unter Verwendung einer ersten semantischen Klasse durch eine Künstliche-Intelligenz-Einrichtung mittels eines Künstliche-Intelligenz-Algorithmus berechnet. Die erste semantische Klasse umfasst zumindest eine Repräsentation einer Abbildung einer anatomischen Struktur, eine Repräsentation einer Abbildung eines medizinischen Implantats, eine Repräsentation einer Abbildung eines medizinischen Werkzeugs oder eine Repräsentation einer Abbildung eines medizinischen Befestigungselementes. Das erste Teilbild und die Bilddaten werden gemischt, um ein Ausgabebild unter Einsatz einer ersten einstellbaren Funktion mit zumindest einem ersten Parameter zu erzeugen, wobei die erste Funktion die Darstellung des ersten Teilbildes im Ausgabebild bestimmt. Der zumindest eine erste Parameter wird durch zumindest eine Benutzereingabe und/oder dem Typ des zu untersuchenden anatomischen Bereichs und/oder der ersten semantischen Klasse und/oder der Art des medizinischen Eingriffs ausgewählt. Das Ausgabebild wird auf einer geeigneten Anzeigevorrichtung ausgegeben.

Zum Trainieren des Künstliche-Intelligenz-Algorithmus der Künstliche-Intelligenz-Einrichtung werden Trainingsdaten erzeugt. Die Trainingsdaten zum Trainieren des Künstliche-Intelligenz-Algorithmus der Künstliche-Intelligenz-Einrichtung weisen zumindest einen Satz aus einem Eingangsbild und einem ersten Zielbild auf. Das erste Zielbild wird durch Ersetzen in einem Bereich zumindest eines Eingangsbildes oder eines Bereichs eines Bildmodells, aus dem das Eingangsbild erzeugt wird, von Bilddaten, die die erste semantische Klasse darstellen, durch Bilddaten, die das umgebende Gewebe darstellen, erzeugt. Jedes Eingangsbild entspricht einem Projektionsbild des zu untersuchenden anatomischen Bereichs, das durch Strahlung erzeugt wird oder dessen Erzeugung durch Strahlung teilweise oder vollständig simuliert wird. Zumindest ein erstes Zielbild weist Bildinformation der ersten semantischen Klasse auf.

Eine Mehrzahl erster Zielbilder kann die erste semantische Klasse aufweisen, und eine zweite Mehrzahl erster Zielbilder kann die erste semantische Klasse nicht aufweisen. Das Bildmodell kann zweidimensional oder dreidimensional sein. Die Künstliche-Intelligenz-Einrichtung kann ein Computer sein, auf dem eine Software läuft, die Rechenoperationen auf Grundlage künstlicher Intelligenz durchführt, beispielsweise mittels eines neuronalen Netzwerkes. Die Trainingsdaten werden zum Trainieren des Künstlichen-Intelligenz-Algorithmus zum Ausführen des Schrittes des Berechnens des ersten Teilbildes durch die Künstliche-Intelligenz-Einrichtung verwendet. Das Projektionsbild kann aus einem dreidimensionalen Bildvolumen erzeugt werden.

Die Darstellung des ersten Teilbildes im Ausgabebild kann zumindest die Sichtbarkeit und/oder die Helligkeit und/oder den Kontrast und/oder die Farbe des ersten Teilbildes im Ausgabebild umfassen.

Der Schritt des Erzeugens der Trainingsdaten für den Künstliche-Intelligenz-Algorithmus der Künstliche-Intelligenz-Einrichtung kann auf einer Künstliche-Intelligenz-Trainingseinrichtung durchgeführt werden. Der Künstliche-Intelligenz-Algorithmus kann auf einem Trainingscomputer oder einem anderen Computer erzeugt werden und auf die Künstliche-Intelligenz-Einrichtung übertragen werden. Es versteht sich, dass das Übertragen auch das Speichern des Künstliche-Intelligenz-Algorithmus in einem permanenten Speicher aufweisen kann. Der Künstliche-Intelligenz-Algorithmus kann auf dem Trainingscomputer oder einem beliebigen anderen Computer mittels der Trainingsdaten trainiert werden.

Jedes Eingangsbild kann Bildinformation der ersten semantischen Klasse aufweisen.

Für den Betrachter von Röntgenbildern sind häufig nur Strukturen im Bild von Interesse, die zu bestimmten semantischen Klassen von Objekten gehören, während Strukturen, die zu anderen semantischen Klassen gehören, den Betrachter stören. Wird Röntgenbildgebung beispielsweise in der minimalinvasiven Gefäßchirurgie eingesetzt, um Interventionsmaterialien wie Führungsdrähte, Katheter oder Stents durch den Patienten zu führen, so möchte der Chirurg hauptsächlich diese Interventionsmaterialien im Röntgenbild sehen. Knochen hingegen möchte der Chirurg nicht, oder nicht mit vollem Kontrast sehen, da sie, falls sie im Röntgenbild mit den Interventionsmaterialien überlagert sind, das Erkennen der letzteren erschweren. In diesem Fall wäre also eine Erhöhung der Sichtbarkeit von Strukturen, die zur semantischen Klasse "Interventionsmaterial" gehören und eine Verringerung der Sichtbarkeit von Strukturen, die zur semantischen Klasse "Knochen" gehören, wünschenswert.

Gleichermaßen ist es bei der Injektion von Röntgen-Kontrastmitteln das Ziel, möglichst nur die semantische Klasse der Blutgefäße sichtbarzumachen, wofür konventionell in Subtraktionsverfahren (DSA) ein Hintergrundbild subtrahiert wird.

Die Erfinder der vorliegenden Erfindung haben sich zum Ziel gesetzt, die Sichtbarkeit bzw. den lokalen Kontrast von Strukturen im Röntgenbild zu erhöhen oder zu verringern, je nachdem zu welcher semantischen Klasse diese Strukturen gehören. Zwar sind viele Methoden bekannt, die die Sichtbarkeit von Strukturen in Röntgenbildern erhöhen oder verringern können, allerdings basieren diese in der Regel nicht auf der Semantik der Strukturen, sondern auf einfacher zu berechnenden, ersatzweise verwendeten Bildmerkmalen, wie der Helligkeit von Pixeln oder der Frequenzdarstellung (Fouriertransformierte) des Bildes.

Beispielsweise erhöht die Anwendung einer Tonwertkurve die Sichtbarkeit von Strukturen, deren Pixelhelligkeiten in einem bestimmten Helligkeitsbereich liegen. Wollte man beispielsweise die Sichtbarkeit von Stents im Bild erhöhen und wüsste man, dass diese meistens dunkelgrau im Bild erschienen, so könnte man durch Anwenden einer geeigneten Tonwertkurve die Sichtbarkeit von dunkelgrauen Strukturen erhöhen. Ein solches Verfahren birgt aber den Nachteil, dass die Sichtbarkeit von Stents, die nicht dunkelgrau erscheinen, nicht erhöht würde und die Sichtbarkeit von dunkelgrauen Strukturen, die keine Stents sind, erhöht würde. Das Merkmal "dunkelgrau" wäre also nur ein Ersatz für das semantische Merkmal "Stent", auf dem die Erhöhung der Sichtbarkeit besser basieren sollte.

Für jede semantische Klasse aus einer zuvor festgelegten Menge von semantischen Klassen wird aus einem Eingangsbild ein Teilbild berechnet, wobei das Teilbild nur diejenigen Strukturen des Eingangsbildes enthalten soll, die zur jeweiligen Klasse gehören. Teilbilder werden so berechnet, dass eine Subtraktion die entsprechenden Strukturen unsichtbar macht.

Die Teilbilder und das Eingangsbild werden neu zusammengemischt, sodass die Sichtbarkeit von Strukturen, die zu bestimmten semantischen Klassen gehören, verringert oder erhöht wird. Das so erhaltene Bild wird im Folgenden Ausgabebild genannt.

Das Verfahren kann ein zweites Teilbild aus den Bilddaten unter Verwendung einer zweiten semantischen Klasse durch die Künstliche-Intelligenz-Einrichtung mittels des Künstlicher-Intelligenz-Algorithmus aufweisen. Die zweite semantische Klasse kann zumindest eine Repräsentation einer Abbildung einer anatomischen Struktur, eine Repräsentation einer Abbildung eines medizinischen Implantats, eine Repräsentation einer Abbildung eines medizinischen Werkzeugs oder eine Repräsentation einer Abbildung eines medizinischen Befestigungselementes aufweisen. Das Verfahren kann ferner das erste Teilbild, das zweite Teilbild und die Bilddaten mischen, um das Ausgabebild unter Einsatz einer Funktion mit einer ersten einstellbaren Funktion mit dem zumindest einem ersten Parameter und mit einer zweiten einstellbaren Funktion mit dem zumindest einen zweiten Parameter zu erzeugen. Der zumindest eine zweite Parameter wird durch zumindest eine Benutzereingabe und/oder den Typ des zu untersuchenden anatomischen Bereichs und/oder der zweiten semantischen Klasse und/oder der Art des medizinischen Eingriffs ausgewählt. Die zweite Funktion bestimmt die Darstellung des zweiten Teilbildes im Ausgabebild.

Die Darstellung des zweiten Teilbildes im Ausgabebild kann zumindest die Sichtbarkeit und/oder die Helligkeit und/oder den Kontrast und/oder die Farbe des zweiten Teilbildes im Ausgabebild umfassen.

Der zumindest eine erste Parameter ist ein Maß, wie deutlich und/oder kontrastreich und/oder in welcher Farbe und/oder mit welcher Helligkeit die erste semantische Klasse in dem Ausgabebild dargestellt wird oder ob sie unterdrückt wird. Der zumindest eine zweite Parameter ist ein Maß, wie deutlich und/oder kontrastreich und/oder in welcher Farbe und/oder mit welcher Helligkeit die zweite semantische Klasse in dem Ausgabebild dargestellt wird oder unterdrückt wird.

In einem Beispiel kann der zu untersuchende anatomischer Bereich der Brustbereich eines Patienten sein. Der Bediener einer medizinischen Bildgebungsvorrichtung kann beispielsweise einen Knochen als erste semantische Klasse definieren und ihn weitestgehend unterdrücken, und einen Stent oder Führungsdraht als zweite semantische Klasse definieren und deren Kontrast im Ausgabebild erhöhen.

Das Verfahren kann ein n-tes Teilbild aus den Bilddaten unter Verwendung einer n-ten semantischen Klasse durch die Künstliche-Intelligenz-Einrichtung mittels des Künstliche-Intelligenz-Algorithmus berechnen. Die n-te semantische Klasse kann eine Repräsentation einer Abbildung einer anatomischen Struktur, eine Repräsentation einer Abbildung eines medizinischen Implantats, eine Repräsentation einer Abbildung eines medizinischen Werkzeugs oder eine Repräsentation einer Abbildung eines medizinischen Befestigungselementes umfassen. Das Verfahren kann die Teilbilder 1 bis n mit den Bilddaten mischen, um das Ausgabebild unter Einsatz einer Funktion mit n (Unter-)Funktionen mit je zumindest einem Parameter zu erzeugen. Der jeweilige Parameter kann durch eine Benutzereingabe und/oder den Typ des zu untersuchenden anatomischen Bereichs und/oder der n-ten semantischen Klasse und/oder der Art des medizinischen Eingriffs ausgewählt werden. Die erste bis n-te Funktion bestimmen die Darstellung des ersten bis n-ten Teilbildes im Ausgabebild. Die Darstellung des n-ten Teilbildes im Ausgabebild kann zumindest die Sichtbarkeit und/oder die Helligkeit und/oder den Kontrast und/oder die Farbe des n-ten Teilbildes im Ausgabebild umfassen. Der jeweilige Parameter ist ein Maß, wie deutlich und/oder kontrastreich und/oder in welcher Farbe und/oder mit welcher Helligkeit die n-ten semantischen Klasse im Ausgabebild sichtbar ist oder ob die n-te semantische Klasse unterdrückt wird.

Der zumindest eine erste Parameter und/oder der zumindest eine zweite Parameter und/oder der zumindest eine n-te Parameter kann automatisch ausgewählt werden, beispielsweise in Abhängigkeit des zu untersuchenden anatomischen Gewebes oder der Art des medizinischen Eingriffs. Beispielsweise kann in einer Ausführungsform eine Bildgebung für die Heilung eines Knochenbruchs den Knochen und die Schrauben kontrastreicher darstellen, wohingegen Weichgewebe weniger kontrastreich dargestellt wird. In einer anderen Ausführungsform kann die Bildgebung für die Positionierung eines Stents Knochen unterdrücken und den Stent oder den Führungsdraht kontrastreicher darstellen. In einer vorteilhaften Ausgestaltung des erfindungsgemäßen Verfahrens kann die Art des zu untersuchenden anatomischen Gewebes oder die Art des medizinischen Eingriffs über die Auswahl eines entsprechenden Organprogramms durch den Bediener einer medizinischen Bildgebungsvorrichtung festgelegt werden.

Das Eingangsbild kann aus einem dreidimensionalen Bildmodell eines Patienten erzeugt werden, beispielsweise mittels einer Projektion, insbesondere einer synthetischen Vorwärtsprojektion.

Der Schritt des Erzeugens der Trainingsdaten für den Künstliche-Intelligenz-Algorithmus der Künstliche-Intelligenz-Einrichtung kann für den Schritt des Berechnens des Zielbildes mittels zumindest einem Satz aus Eingangsbild und einem oder mehreren Zielbildern den Schritt des Auswählens einer semantischen Klasse als ausgewählte Klasse, deren Bildinformation in einem der Zielbilder enthalten sein soll, umfassen. Das Verfahren lädt ein erstes dreidimensionales Bildmodell des Gewebes eines Patienten in den Speicher des Computers oder der Recheneinheit, wobei das erste dreidimensionale Bildmodell des Gewebes des Patienten dreidimensionale Bildinformation der ausgewählten semantischen Klasse aufweist. Das Verfahren erzeugt ein zweites dreidimensionales Bildmodell, das keine Bildinformation der ausgewählten semantischen Klasse aufweist, durch Ersetzen in einem Bereich des ersten dreidimensionalen Bildmodells des Gewebes des Patienten von Bildinformationen, die die ausgewählte semantischen Klasse darstellen, durch Bildinformationen, die das umgebende Gewebe darstellen. Das Eingangsbild wird aus dem ersten dreidimensionalen Bildmodell des Gewebes erzeugt. Aus dem zweiten dreidimensionalen Bildmodell wird ein Zwischenbild erzeugt. Das Zielbild wird durch Subtrahieren des Zwischenbildes vom Eingangsbild erzeugt.

Das Eingangsbild und das Zwischenbild können durch eine Vorwärtsprojektion, beispielsweise eine synthetische Vorwärtsprojektion erzeugt werden. Das dreidimensionale Bildmodell kann ein synthetisches Bildmodell sein.

Das dreidimensionale Bildmodell des Gewebes des Patienten kann aus einer dreidimensionalen Röntgenbildgebung, beispielsweise einer Computertomographie erzeugt werden. Bei einer anderen Ausführungsform kann das dreidimensionale Bildmodell des Gewebes eines Patienten mittels einer Simulation einer dreidimensionalen Röntgenbildgebung erzeugt werden. Bei einer anderen Ausführungsform kann das dreidimensionale Bildmodell des Gewebes eines Patienten mittels eines statistischen Modells von Patientengewebe erzeugt werden.

Die dreidimensionale Bildinformation (beispielsweise ein dreidimensionales Bildmodell) der ausgewählten semantischen Klasse kann mittels einer dreidimensionalen Röntgenbildgebung erzeugt werden. Bei einer anderen Ausführungsform kann die dreidimensionale Bildinformation der ausgewählten semantischen Klasse mittels einer Simulation einer dreidimensionalen Röntgenbildgebung erzeugt werden.

Der erste Parameter kann mittels einer ersten Bedieneinrichtung eingestellt werden und/oder der zweite Parameter kann mittels einer zweiten Bedieneinrichtung und/oder der n-te Parameter kann mittels einer n-ten Bedieneinrichtung eingestellt werden. Die erste Bedieneinrichtung, die zweite Bedieneinrichtung und/oder die n-te Bedieneinrichtung kann den Kontrast der Bildinformation variieren, die die jeweilige semantische Klasse darstellt.

Soll die Künstliche-Intelligenz-Einrichtung ein zweites Teilbild aus den Bilddaten berechnen, so muss der Künstliche-Intelligenz-Algorithmus mittels entsprechender Trainingsdaten trainiert werden. Vorzugsweise bestehen diese Trainingsdaten aus mindestens einem Satz bestehend aus einem Eingangsbild und zwei Zielbildern. Das erste Zielbild enthält dabei nur solche Bildinformation aus dem Eingangsbild, die zur ersten semantischen Klasse gehören. Das zweite Zielbild enthält dabei nur solche Bildinformation aus dem Eingangsbild, die zur zweiten semantischen Klasse gehören.

Das erste mindestens eine Zielbild wird so berechnet wie zuvor beschrieben, also durch Ersetzen von Bildinformationen im Eingangsbild oder in einem Bildmodell aus dem das Eingangsbild erzeugt wird, die zur ersten semantischen Klasse gehören. Das zweite Zielbild kann analog erzeugt werden, also durch Ersetzen von Bildinformationen im Eingangsbild oder in einem Bildmodell aus dem das Eingangsbild erzeugt wird, die zur zweiten semantischen Klasse gehören.

Das zweite Zielbild kann aber auch auf andere Weise erzeugt werden. Wäre die zweite semantische Klasse beispielsweise "Stent", so könnte als zweites Zielbild die synthetische Vorwärtsprojektion eines simulierten 3D-Modells eines Stents verwendet werden. Hierbei müsste das Eingangsbild natürlich auch die Vorwärtsprojektion des Stents enthalten. Dies könnte erreicht werden, indem man als Eingangsbild die Summe aus dem zweiten Zielbild und einem Projektionsbild, das einen Patienten ohne Stents zeigt, verwendet. Zu beachten ist, dass es in diesem Beispiel zwar zwei Zielbilder gibt, aber nur ein Eingangsbild. Das heißt, das Eingangsbild, das durch Addition erzeugt wurde, ist dasselbe Bild wie das Eingangsbild, in dem die Bildinformationen, die zur ersten semantischen Klasse gehören, ersetzt wurden.

Das Vorgehen bei der Erweiterung von zwei Teilbildern auf n Teilbilder erfolgt analog zum oben beschriebenen Vorgehen bei der Erweiterung von einem Teilbild auf zwei Teilbilder.

Der erste Parameter und/oder der zweite Parameter und/oder der n-te Parameter können ein Gewichtungsfaktor sein.

In einem Ausführungsbeispiel wird das Ausgabebild wie folgt berechnet: 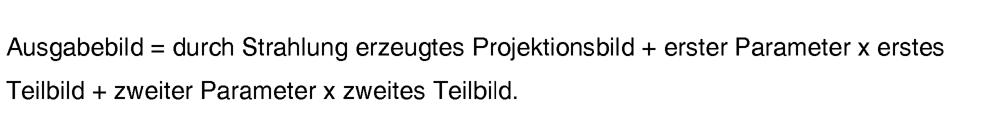 Ausgabebild = durch Strahlung erzeugtes Projektionsbild + erster Parameter x erstes Teilbild + zweiter Parameter x zweites Teilbild.

Die Erfindung betrifft auch ein Computerprogrammprodukt, das, wenn es in einen Speicher eines Computers oder einer Recheneinheit mit einem Prozessor geladen wird, die zuvor beschriebenen Schritte ausführt.

Die Erfindung offenbart auch eine medizinische Bildgebungsvorrichtung mit einer Strahlungsquelle, die Strahlung emittiert, und einen Strahlungssensor, auf den die Strahlung auftrifft, nachdem sie ein anatomisches Gewebe passiert hat, wobei der Strahlungssensor Strahlungsdaten ausgibt. Der Strahlungssensor ist vorzugsweise ein Flachdetektor. Die medizinische Bildgebungsvorrichtung umfasst eine Projektionsbilderzeugungseinrichtung, die aus Strahlungsdaten ein Projektionsbild erzeugt. Die medizinische Bildgebungsvorrichtung umfasst einen Speicher, in den Bilddaten, die das durch Strahlung erzeugte Projektionsbild repräsentieren, geladen werden. Die medizinische Bildgebungsvorrichtung umfasst ferner eine Künstliche-Intelligenz-Einrichtung, die zum Berechnen eines ersten Teilbildes aus den Bilddaten unter Verwendung einer ersten semantischen Klasse mittels eines Künstliche-Intelligenz-Algorithmus ausgebildet ist. Die Künstliche-Intelligenz-Einrichtung ist vorzugsweise derart ausgestaltet, dass sie zur Reduktion der Berechnungszeit eine massiv-parallele Rechenarchitektur enthält, wie beispielsweise einen Grafikprozessor (GPU). Die erste semantische Klasse umfasst zumindest eine Repräsentation einer Abbildung einer anatomischen Struktur, eine Repräsentation einer Abbildung eines medizinischen Implantats, eine Repräsentation einer Abbildung eines medizinischen Werkzeugs oder eine Repräsentation einer Abbildung eines medizinischen Befestigungselementes. Die Künstliche-Intelligenz-Einrichtung umfasst ferner eine Mischeinrichtung, die zum Mischen des ersten Teilbildes und der Bilddaten ausgebildet ist, um ein Ausgabebild unter Einsatz einer ersten einstellbaren Funktion mit zumindest einem ersten Parameter zu erzeugen. Der zumindest eine erste Parameter wird durch zumindest eine Benutzereingabe und/oder dem Typ eines zu untersuchenden anatomischen Bereichs und/oder der ersten semantischen Klasse und/oder der Art des medizinischen Eingriffs ausgewählt. Die medizinische Bildgebungsvorrichtung umfasst ferner eine Anzeigevorrichtung, die zum Ausgeben des Ausgabebildes ausgebildet ist.

Eine Künstliche-Intelligenz-Trainingseinrichtung ist dazu ausgebildet, Trainingsdaten zum Trainieren des Künstliche-Intelligenz-Algorithmus der Künstliche-Intelligenz-Einrichtung mittels mindestens einem Satz aus einem Eingangsbild und einem oder mehreren Zielbildern zu erzeugen. Die Künstliche-Intelligenz-Trainingseinrichtung ist dazu ausgebildet, ein Zielbild durch Ersetzen in einem Bereich zumindest eines Eingangsbildes oder eines Bereichs eines Bildmodells zu erzeugen, aus dem das Eingangsbild erzeugt wird, mittels Bilddaten, die die erste semantische Klasse darstellen, durch Bilddaten, die das umgebende Gewebe darstellen. Jedes Eingangsbild entspricht einem Projektionsbild eines zu untersuchenden anatomischen Bereichs, das durch Strahlung erzeugt wird oder dessen Erzeugung durch Strahlung teilweise oder vollständig simuliert wird. Zumindest ein Zielbild weist Bildinformation der ersten semantischen Klasse auf.

Die medizinische Bildgebungsvorrichtung kann so weitergebildet sein, wie zuvor hinsichtlich des Verfahrens beschrieben wurde.

Die medizinische Bildgebungsvorrichtung kann einen schwenkbaren C-Bogen umfassen. Aus den Strahlungsdaten kann ein dreidimensionales Bildmodell erstellt werden (Computertomografie).

Die Künstliche-Intelligenz-Trainingseinrichtung muss nicht Teil der medizinische Bildgebungsvorrichtung sein. Auf der Künstliche-Intelligenz-Trainingseinrichtung werden Trainingsdaten zum Trainieren des Künstliche-Intelligenz-Algorithmus erzeugt, wobei der trainierte Künstliche-Intelligenz-Algorithmus in die Künstliche-Intelligenz-Einrichtung der medizinische Bildgebungsvorrichtung geladen und dort gespeichert wird.

Das Erzeugen der Trainingsdaten für den Künstliche-Intelligenz-Algorithmus kann dabei auf der Künstliche-Intelligenz-Trainingseinrichtung selbst erfolgen oder auf einer anderen hierfür geeigneten Einrichtung, wobei im zweiten Fall die Trainingsdaten anschließend der Künstliche-Intelligenz-Trainingseinrichtung zugeführt werden. Dies ist insbesondere dann vorteilhaft, wenn die Einrichtung für die Erzeugung der Trainingsdaten und die Künstliche-Intelligenz-Trainingseinrichtung zur Beschleunigung der Recheneffizienz in unterschiedlicher Weise an ihre Aufgabe angepasst sind.

### Kurze Beschreibung der Figuren

Die Erfindung wird nun unter Bezugnahme auf die Figuren, die nicht einschränkende Ausführungsformen der Erfindung zeigen, detaillierter beschrieben, wobei:
Figur 1 eine medizinische Bildgebungsvorrichtung zeigt;
Figur 2 schematisch die Funktionsweise des Verfahrens und der Vorrichtung der vorliegenden Erfindung darstellt;
Figur 3 zeigt schematische Darstellung von beispielhaften Eingangsbildern, Teilbildern und Ausgabebildern in der Anwendung eines bereits trainierten Künstliche-Intelligenz-Algorithmus;
Figur 4 schematisch das Trainieren einer Künstlichen-Intelligenz-Einrichtung darstellt, falls eine semantische Klasse trainiert werden soll;
Figur 5 schematisch das Trainieren einer Künstlichen-Intelligenz-Einrichtung darstellt, falls mehrere semantische Klassen trainiert werden sollen; und
Figur 6 verschiedene Möglichkeiten zum Zusammenmischens des Eingangsbildes und Teilbildern zeigt.

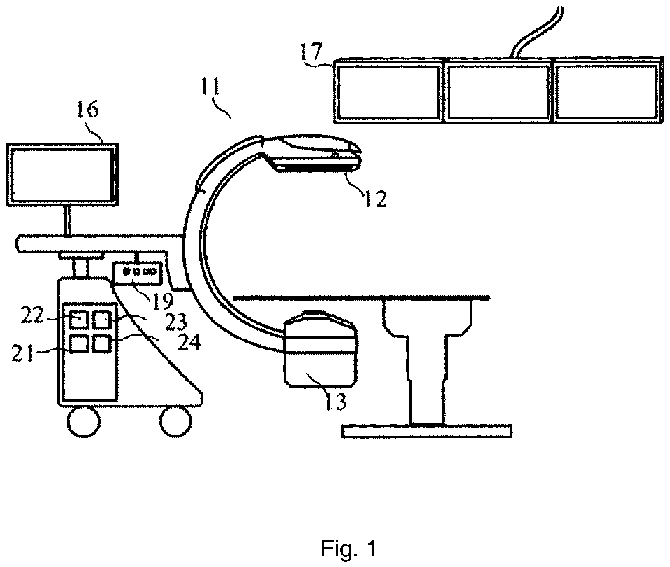

In Figur 1 ist eine erfindungsgemäße Vorrichtung in Ausgestaltung eines mobilen C-Bogens 11 schematisch dargestellt, welcher für die Ausführung des trainierten Künstliche-Intelligenz-Algorithmus mittels der Künstliche-Intelligenz-Einrichtung des erfindungsgemäßen Verfahrens vorgesehen ist.

Der C-Bogen 11 trägt an seinem ersten Ende einen Röntgengenerator 13, und an seinem zweiten Ende und dem Röntgengenerator 13 gegenüberliegend einen Röntgenbilddetektor 12, beispielsweise kann es sich hierbei um einen Flachdetektor oder einen Bildverstärker handeln. Der C-Bogen 11 ist in mehreren Achsen im Raum motorisch gesteuert oder manuell verstellbar, wobei die Achsen Sensoren zur Erfassung des Maßes der Verstellung aufweisen können.

Ferner beinhaltet die Vorrichtung eine Bildverarbeitungseinheit 21, eine Speichereinheit 22, einer Steuerungseinheit 23, sowie eine Netzwerkschnittstelle 24. Mittels der Netzwerkschnittstelle 24 können Daten, beispielsweise Bilddatensätze und Resultate des erfindungsgemäßen Verfahrens, in einem Netzwerk verteilt bzw. zur Verfügung gestellt werden. Die Bildverarbeitungseinheit 21 kann eine Künstliche-Intelligenz-Einrichtung aufweisen.

Die Bildverarbeitungseinheit 21 umfasst eine Speichereinheit 22, auf dieser können die für das erfindungsgemäße Verfahren verwendeten zwei- oder dreidimensionalen Bilddatensätze gespeichert bzw. geladen werden. Diese Bilddatensätze können entweder von einem Server geladen oder mittels des C-Bogen 11 vor, während oder nach einer Intervention aufgenommen werden. Ferner umfasst die Speichereinheit 22 Instruktionen, die für die Ausführung des erfindungsgemäßen Verfahrens mittels der Bildverarbeitungseinheit 21 verwendet werden.

Ferner kann die Vorrichtung eine graphische Benutzeroberfläche, mit einer Bildausgabeeinheit 16, 17 und einer Eingabeeinheit 19 umfassen, mit welchen für die Bildverarbeitungseinheit 21 entsprechende Einstellungen in entsprechenden Organprogrammen vorgenommen werden können.

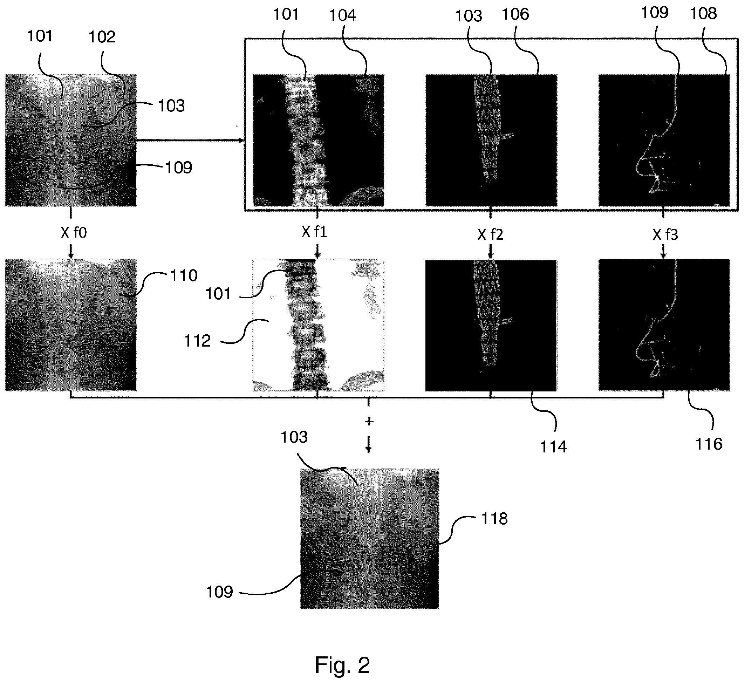

Figur 2 visualisiert das erfindungsgemäße Verfahren. Das Eingangsbild 102 zeigt einen Knochen 101, einen Stent 103 und einen Führungsdraht 109. Im ersten Schritt werden aus einem Röntgenbild, dem Eingangsbild 102, Teilbilder 104, 106, 108 mittels des Künstliche-Intelligenz-Algorithmus berechnet, der auf einer Künstliche-Intelligenz-Einrichtung läuft. Jedes Teilbild 104, 106, 108 beinhaltet die Strukturen einer bestimmten semantischen Klasse. Das Teilbild 104 ist das Teilbild für die semantische Klasse Knochen 101, das Teilbild 106 ist das Teilbild für die semantische Klasse Stent 103, und das Teilbild 108 ist das Teilbild für die semantische Klasse Führungsdraht 109.

Im zweiten Schritt werden das Eingangsbild 102 und die Teilbilder 104, 106, 108 gemischt. Im Ausgabebild 118 ist dann die Sichtbarkeit von Strukturen bestimmter semantischer Klassen verringert, beispielsweise der Knochen 101 oder erhöht, beispielsweise des Stents 103 und der Führungsdrähte 109 Das Zusammenmischen erfolgt in dieser Ausführungsform durch eine Linearkombination. Das Eingangsbild 102 und die Teilbilder 104, 106, 108 werden mit Gewichtungsfaktoren multipliziert und ergeben ein gewichtetes Eingangsbild 110 und gewichtete Teilbilder 112, 114, 116. Anschließend werden das gewichtete Eingangsbild 110 und die gewichteten Teilbilder 112, 114, 116 addiert.

Die Formel für das Mischen des Eingangsbildes 102 und der Teilbilder 104, 106, 108 lautet in dieser Ausführungsform wie folgt: 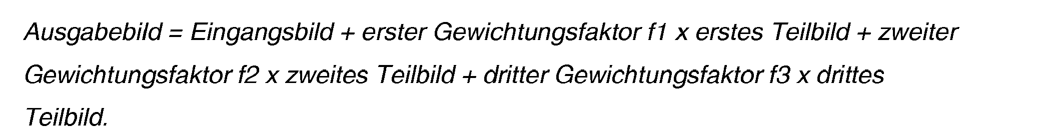 Ausgabebild = Eingangsbild + erster Gewichtungsfaktor f1 x erstes Teilbild + zweiter Gewichtungsfaktor f2 x zweites Teilbild + dritter Gewichtungsfaktor f3 x drittes Teilbild.

Die Gewichtungsfaktoren können automatisch eingestellt werden oder mittels eines Bedienelementes eingestellt werden.

Es ist möglich, Teilbilder 104, 106, 108 farblich darzustellen, wodurch Strukturen 101, 103, 109 farblich hervorgehoben werden können.

Das erfindungsgemäße Verfahren kann in Quasi-Echtzeit angewendet werden, d.h. Röntgenbilder 102 werden sofort nach Aufnahme und eventueller Vorverarbeitung durch das erfindungsgemäße Verfahren bearbeitet und das Ausgabebild 118 dem Betrachter angezeigt. Weiterhin ist es denkbar, das Verfahren auf ältere Eingangsbilder 102, welche beispielsweise aus einem Patientenarchiv geladen werden, anzuwenden und die Ausgabebilder anzuzeigen. Die Schritte müssen außerdem nicht gleich oft ausgeführt werden. Insbesondere kann es bezüglich der benötigten Rechenzeit vorteilhaft sein, die Teilbilder 104, 106, 108 nur einmal zu berechnen (Schritt 1) und diese anschließend mehrfach zusammenzumischen (Schritt 2), beispielsweise um sich der gewünschten Mischung iterativ anzunähern oder um verschiedene Mischungen, die verschiedene Bildaspekte hervorheben, getrennt zu betrachten.

Beim Eingangsbild 102 handelt es sich um ein Röntgenbild. Vorzugsweise wird kein rohes Röntgenbild, d.h. so wie es der Detektor gemessen hat, verwendet, sondern ein vorverarbeitetes Röntgenbild. Standardvorerarbeitungsschritte sind beispielsweise eine Offsetkorrektur, eine Gainkorrektur, eine Defekte-Pixel-Korrektur, eine Flatfield-Korrektur bzw. eine I₀-Normierung, ein Logarithmieren, eine Wasservorkorrektur, eine Streustrahlkorrektur, eine Anwendung von Tonwertkurven, Anwendung von Hoch- oder Tiefpässen, eine Anwendung temporaler Filter etc.

Die Wahl der semantischen Klassen orientiert sich sinnvollerweise an den aktuellen Bedürfnissen des Betrachters. Denkbar ist beispielsweise, dass der Betrachter die semantischen Klassen, deren Sichtbarkeit er verändert sehen möchte, frei aus der Menge aller semantischen Klassen, für die die Berechnung der entsprechenden Teilbilder implementiert ist, auswählen kann. Weiterhin denkbar ist die Festlegung der semantischen Klassen durch das aktuell ausgewählte Organprogramm, d.h. dem zu untersuchenden anatomischen Bereich und/oder der Art des Eingriffs. In einem Organprogramm für gefäßchirurgische Eingriffe wären beispielsweise die semantischen Klassen Blutgefäße, Knochen, Stent, Führungsdraht und Katheter sinnvoll. In einem Organprogramm für orthopädische Eingriffe wären beispielsweise die Klassen Knochen, Schrauben und Verbindungsstangen sinnvoll.

Damit das Eingangsbild 102 und die Teilbilder 104, 106, 108 in Schritt 2 so zusammengemischt werden können, dass das Ausgabebild 118 den Bedürfnissen des Betrachters entspricht, sind die genauen Pixelwerte der Teilbilder 104, 106, 108 von Bedeutung. Insbesondere sollen die Teilbilder nach der Maßgabe berechnet werden, dass eine einfache Subtraktion eines Teilbilds 104, 106, 108 vom Eingangsbild 102 ein Ausgabebild produziert, in dem die Strukturen der semantischen Klasse des Teilbildes 104, 106, 108 möglichst vollständig unterdrückt sind. Die Subtraktion ist ein Spezialfall des in Schritt 2 erfolgenden Zusammenmischens.

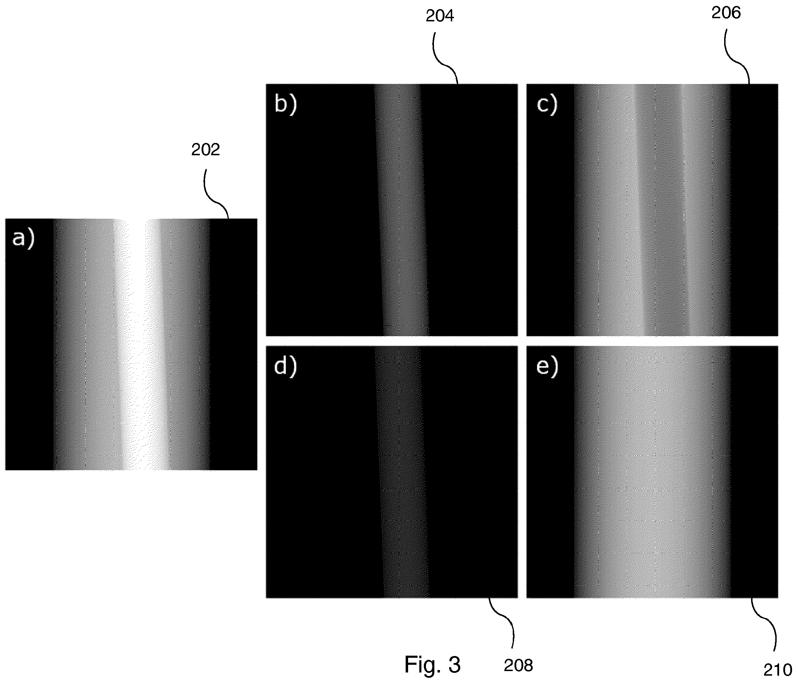

Ein physikalisch motivierter Ansatz sähe vor, die Teilbilder 104, 106, 108 so zu berechnen, dass die Teilbilder den physikalischen Beitrag der Strukturen der jeweiligen semantischen Klasse zum Eingangsbild enthalten. Solche Teilbilder sind aber nicht zur o.g. Subtraktion geeignet, denn es blieben nach Subtraktion eines Teilbilds die Strukturen der entsprechenden semantischen Klasse als "Negativabdruck", d.h. mit invertiertem Kontrast, im Ausgabebild 118 sichtbar. Dieser Effekt ist in Figur 3 am Beispiel eines Knochen-Teilbilds schematisch dargestellt. Wird vom Eingangsbild 202 das physikalische Knochen-Teilbild 204 subtrahiert, so zeigt das erste Ausgabebild 206 in unerwünschter Weise immer noch die Knochen, jedoch mit invertiertem Kontrast. Dieser Effekt ist leicht verständlich, wenn man bedenkt, dass das Eingangsbild nur aus den Beiträgen von Weichgewebe und Knochen zusammengesetzt ist. Entfernt man nun den Knochenbeitrag durch Subtraktion des physikalischen Knochen-Teilbildes, bleibt nur der Weichgewebsbeitrag im Ausgabebild übrig, am Ort des Knochens verbleibt entsprechend ein leerer Bereich.

Da sich im Weichgewebe des Patienten dann aber knochenförmige Hohlräume befinden, sind diese knochenförmigen Hohlräume im Ausgabebild sichtbar, insbesondere weil sich kein Weichgewebe dort befinden kann, wo sich Knochen befindet.

Um diesen Effekt zu vermeiden, wird jedes der Teilbilder erfindungsgemäß unter der Maßgabe berechnet. dass nach Subtraktion eines Teilbilds vom Eingangsbild die Strukturen der jeweiligen semantischen Klasse im Ausgabebild tatsächlich unsichtbar sind. Dies unterscheidet die vorliegende Erfindung von den oben beschriebenen physikalisch motivierten Ansätzen des Standes der Technik. Ein so berechnetes Knochen-Teilbild 208 ist in Figur 3 zu sehen. Nach Subtraktion dieses Knochen-Teilbildes vom Eingangsbild 202 sind die Knochen im zweiten Ausgabebild 210 unsichtbar.

Figur 3 zeigt schematische Darstellung von beispielhaften Eingangsbildern, Teilbildern und Ausgabebildern in der Anwendung eines bereits trainierten Künstliche-Intelligenz-Algorithmus. In diesem Beispiel ist "Knochen" die einzige semantische Klasse. Das Eingangsbild 202 ist ein vereinfachtes Modell eines Röntgenbildes eines Oberschenkels, wobei der Knochen als weißer Bereich im Inneren dargestellt ist. Das Knochen-Teilbild 204 wurde nach dem physikalischen Ansatz berechnet, wonach das Knochen-Teilbild 204 dem physikalischen Beitrag der Knochen zum Eingangsbild gleicht. Im ersten Ausgabebild 206, das man durch Subtraktion des Knochen-Teilbildes 204 von Eingangsbild 202 erhält, sind die Knochen noch sichtbar, jedoch mit invertiertem Kontrast, während die Knochen im Eingangsbild 202 heller als die Umgebung sind, sind sie im ersten Ausgabebild 206 dunkler. Das erfindungsgemäße Knochen-Teilbild 208 ist so berechnet worden, dass nach Subtraktion des Knochen-Teilbilds 208 vom Eingangsbild 202 die Knochen unsichtbar sind. Im zweiten Ausgabebild 210, das man durch Subtraktion des erfindungsgemäßen Knochen-Teilbildes 208 vom Eingangsbild 202 erhält, sind die Knochen unsichtbar. Dies entspricht eher der Erwartung menschlicher Betrachter als im ersten Ausgabebild 206.

Die Berechnung der Teilbilder 104, 106, 108 erfolgt idealerweise durch ein Maschinenlernverfahren (MLV), beispielsweise ein künstliches neuronales Netz (NN), vorzugsweise durch ein Convolutional Neural Network (CNN), dessen Input das Eingangsbild ist und dessen Outputs die Teilbilder sind. Obwohl im Folgenden "MLV" in der Einzahl verwendet wird, kann es vorteilhaft sein mehrere MLVs zu verwenden, die jeweils nur ein Teilbild 104, 106, 108 oder mehrere Teilbilder 104, 106, 108 ausgeben. Beispielsweise könnte die Berechnung der Teilbilder genauer oder effizienter sein, wenn für jede semantische Klasse ein separates MLV verwendet würde.

Bevor ein MLV seine Aufgabe erfüllen kann, muss es trainiert werden. Dies kann beispielsweise durch überwachtes Training erfolgen. In einem überwachten Training lernt ein MLV aus einer Vielzahl von Eingangselement-Ausgangselement-Sätzen (Input-Output-Sätzen). Jeder Satz besteht aus einem Eingangselement und einem Ausgangselement. Im Kontext dieser Erfindung wäre das Eingangselement eines solchen Satzes ein Eingangsbild. Das Ausgangelement des Satzes wären ein dem Eingangsbild zugehöriges Zielbild oder mehrere dem Eingangsbild zugehörige Zielbilder. Unter einem, dem Eingangsbild zugehörigen Zielbild wird hierbei ein möglichst ideales, dem Eingangsbild zugehöriges Teilbild verstanden, also ein Teilbild, das das MLV idealerweise ausgäbe, wenn es als Input das Eingangsbild erhielte. Da MLVs in der Regel nicht perfekt funktionieren, unterscheiden sich Teilbilder (was das MLV tatsächlich ausgibt) und Zielbilder (was das MLV ausgeben soll).

Man würde zunächst erwarten, die für ein erfolgreiches Training nötige Anzahl an Eingangselement-Ausgangselement-Sätzen durch echte klinische Daten in Form von Röntgenprojektionen (2D) erhalten zu können. Zum einen sind diese jedoch schwer zu erhalten (Datenschutzrichtlinien, Zustimmung der Patienten, Verträge mit Kliniken etc.). Zum anderen lassen sich aus diesen aber auch keine Zielbilder gewinnen, da die Beiträge der den Zielbildern zugehörigen semantischen Klassen mit den Beiträgen anderer Strukturen in den Röntgenbildern überlagert sind. In anderen Worten ließe sich hier kein Ausgangselement mit vertretbarem Aufwand erstellen, wodurch das Training des MLV nicht durchgeführt werden kann.

Die Erfinder der vorliegenden Erfindung haben erkannt, dass es vielversprechender ist, die Eingangselement-Ausgangselement-Sätze zumindest in Teilen durch realistische Simulation zu erzeugen. Eine Simulation eines Eingangselement-Ausgangselement-Satzes könnte beispielsweise auf einem 3D-Modell eines Patienten, der keine Objekte der semantischen Klassen enthält, und 3D-Modellen von Objekten der semantischen Klassen basieren. Unter 3D-Modell wird hier jede dreidimensionale Repräsentation eines Objekts verstanden, auch echte Patientendaten. Beispielsweise sind Voxel-Volumina (also auch Computertomographien), Dreiecksgitter und CAD-Modelle, 3D-Modelle. Durch Kombinieren aller 3D Modelle (Patient und Objekte der semantischen Klassen) würde man ein Gesamtmodell erhalten. Als Eingangsbild kann dann ein simuliertes Röntgenbild, erzeugt durch synthetische Vorwärtsprojektion (Digitally Reconstructed Radiograph (DRR)) des Gesamtmodells, dienen.

Ein zugehöriges Zielbild kann erzeugt werden, indem vom Eingangsbild ein fiktives Eingangsbild subtrahiert wird, wobei das fiktive Eingangsbild durch synthetische Vorwärtsprojektion eines fiktiven Gesamtmodells erzeugt wird. Das fiktive Gesamtmodell wird dabei erzeugt, indem im Gesamtmodell die Objekte der (dem zu erzeugenden Zielbild entsprechenden) semantischen Klasse durch die umgebenden Materialien ersetzt werden.

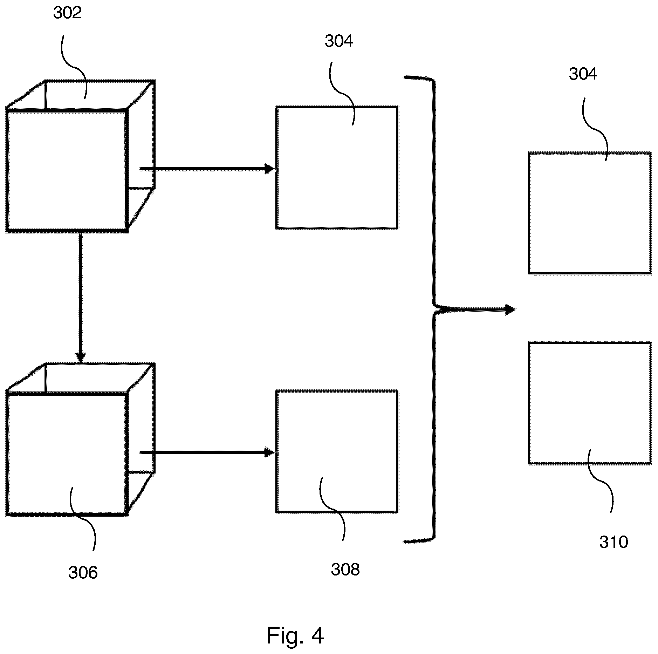

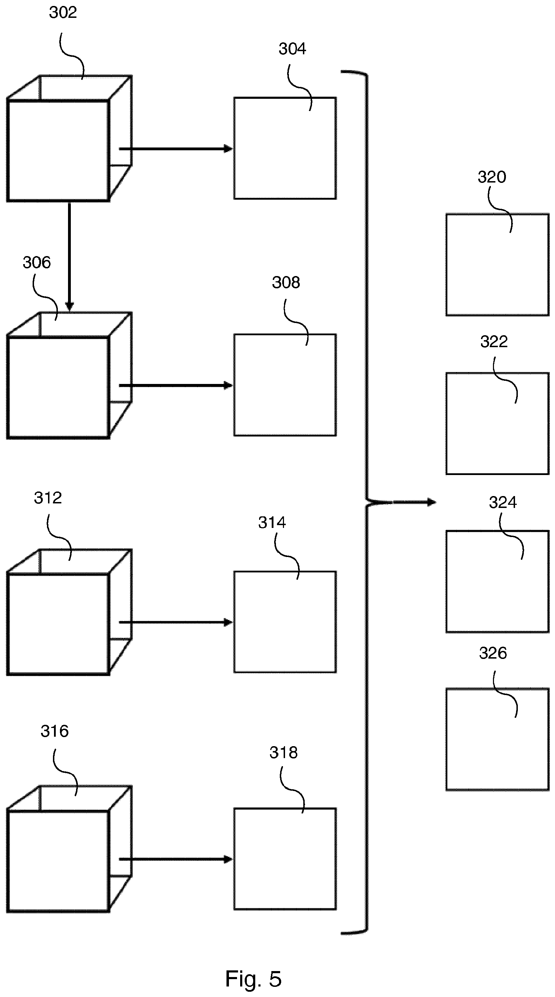

Die Vorgehensweise zum Simulieren von Eingangselement-Ausgangselement-Sätzen ist in den Figuren 4 und 5 schematisch dargestellt. Die in diesem Absatz beschriebene Simulation von Eingangsbildern und Zielbildern ist nur zum Training des MLVs, nicht aber während seiner Anwendung nötig. Sie stellt sicher, dass nach Subtraktion eines Teilbilds bzw. Zielbilds vom Eingangsbild kein Negativabdruck der Strukturen der semantischen Klasse (wie im ersten Ausgabebild 206 entsteht, sondern die Strukturen der semantischen Klasse tatsächlich unsichtbar sind, wie im zweiten Ausgabebild 210).

Die 3D-Modelle, auf denen die oben beschriebene Simulation der Trainingsdaten basiert, können beispielsweise durch Simulation oder aus CT-Volumen gewonnen werden. Ein 3D-Modell eines Patienten kann beispielsweise aus einem klinischen CT-Volumen eines Patienten erzeugt werden. Durch Segmentierung können außerdem 3D-Modelle von Knochen oder von Kontrastmittel-haltigen Gefäßen aus einem CT-Volumen erzeugt werden. Weiterhin können 3D-Modelle von Objekten von semantischen Klassen durch geeignete Simulation erzeugt werden. Beispielsweise kann ein Führungsdraht als gekrümmter Zylinder, dessen Mittellinie durch einen Polynomzug mit zufällig gewählten Kontrollpunkten gegeben ist, modelliert werden. Durch Kombinieren einer Vielzahl von 3D-Modellen und durch Berechnung einer Vielzahl von DRRs pro Gesamtmodell kann ein großer Trainingsdatensatz erzeugt werden. Ist das MLV mit diesem Trainingsdatensatz einmal trainiert, kann es auf beliebige Röntgenbilder angewendet werden. Das Vorhandensein von 3D-Modellen ist dann nicht mehr erforderlich.

Figur 4 zeigt beispielhafte Erzeugung von Trainingsdaten, wenn die künstliche Intelligenz auf nur eine semantische Klasse trainiert werden soll. In diesem Beispiel handelt es sich bei der semantischen Klasse um "Knochen".

Das Eingangsbild 304 ist eine Vorwärtsprojektion, beispielsweise eine synthetische Vorwärtsprojektion, eines Patienten-Modells 302. Das Eingangsbild enthält also insbesondere Weichgewebe und Knochen.

Zur Erzeugung des Knochen-Zielbildes 310 werden zunächst alle Knochen im Patienten-Modell 302 durch Weichgewebe ersetzt, um ein knochenlosen Patienten-Modell 306 zu erzeugen. Von diesem knochenlosen Patienten-Modell 306 wird anschließend eine Vorwärtsprojektion 308, beispielsweise eine synthetische Vorwärtsprojektion, berechnet, die ein erstes fiktives Eingangsbild 308 erzeugt. Die Vorwärtsprojektion 308 entspricht also dem ersten fiktiven Eingangsbild, d.h. dem Eingangsbild, in dem die Knochen durch Weichgewebe ersetzt wurden. Das Knochen-Teilbild 310 ist dann die Differenz aus Eingangsbild 304 und fiktivem Eingangsbild 308.

Das Eingangsbild 304 und das Knochen-Teilbild 310 werden zum Training der künstlichen Intelligenz verwendet. Das Training einer künstlichen Intelligenz ist dem Fachmann bekannt.

Insbesondere existieren hierfür öffentlich verfügbare Software-Bibliotheken wie beispielsweise Tensorflow oder PyTorch, die verschiedene moderne Künstliche-Intelligenz-Algorithmen und entsprechende Architekturen bereitstellen, beispielsweise Convolutional Neural Networks, wie beispielsweise sog. U-Nets.

Diese können dann in vergleichsweise einfacher Weise in Programmiersprachen wie beispielsweise C++ oder Python eingebunden werden. Ferner stellen diese Bibliotheken umfangreiche Hilfsmittel zur Verfügung, um ein Trainieren der Künstliche-Intelligenz-Algorithmen bzw. der Künstliche-Intelligenz-Einrichtung möglichst einfach zu gestalten, z.B. durch Replikation und Anpassung von Trainingsdaten durch Drehung, Spiegelung, Kontraständerung usw.

Das Training des Künstliche-Intelligenz-Algorithmus kann beispielsweise durch ein überwachtes Training erfolgen. In einem überwachten Training wird dem Künstliche-Intelligenz-Algorithmus mindestens ein Eingabe-Ausgabe-Satz präsentiert. In unserem Fall besteht der Eingabe-Teil eines solchen Satzes aus dem Eingangsbild. In unserem Fall besteht der Ausgabe-Teil eines solchen Satzes aus den Zielbildern.

Figur 5 zeigt beispielhaft die Erzeugung von Trainingsdaten, wenn die künstliche Intelligenz auf mehrere semantische Klassen trainiert werden soll. In diesem Beispiel handelt es sich bei den semantischen Klassen um "Knochen", "Stent", "Führungsdraht".

Das Eingangsbild 320 wird erzeugt, indem zur Vorwärtsprojektion des Patienten-Modells 304, eine Vorwärtsprojektion eines Stent-Modells 314 und eine Vorwärtsprojektion eines Führungsdraht-Modells 318 addiert werden. Das Eingangsbild 320 enthält also insbesondere Weichgewebe, Knochen, sowie zumindest einen Stent und zumindest einen Führungsdraht.

Zur Erzeugung des knochenlosen Patienten-Modells306 werden zunächst alle Knochen im Patienten-Modell durch Weichgewebe ersetzt, wie unter Bezugnahme auf Figur 4 beschrieben wurde. Das Knochen-Zielbild 322 wird durch Subtraktion einer Vorwärtsprojektion 308 des knochenlose Patienten-Modells 306 von einer Vorwärtsprojektion 304 des Patienten-Modells 302 erzeugt, wie unter Bezugnahme auf Figur 4 beschrieben wurde.

Das Stent-Zielbild 314 wird durch eine Vorwärtsprojektion aus einem dreidimensionalen Stent-Bildmodell 312 erzeugt, das einen oder mehrere Stents aufweist. Das Führungsdraht-Zielbild 318 wird durch eine Vorwärtsprojektion aus einem dreidimensionalen Führungsdraht-Bildmodell 316 erzeugt, das einen Führungsdraht oder mehrere Führungsdrähte aufweist.

Das Eingangsbild 320 und die Zielbilder 322, 324, 326 werden später zum Training der Künstlichen-Intelligenz-Einrichtung und/oder des Künstlichen-Intelligenz-Algorithmus verwendet. Von den mehreren Zielbildern 322, 324, 326 (bei dieser Ausführungsform werden drei Zielbilder verwendet) wird mindestens ein Zielbild 322 (bei dieser Ausführungsform das Knochen-Zielbild 322) durch das Ersetzungs-Subtraktions-Verfahren erzeugt. Die anderen Zielbilder 324, 326 können anders erzeugt werden (in diesem Beispiel durch Vorwärtsprojektion entsprechender 3D-Modelle).

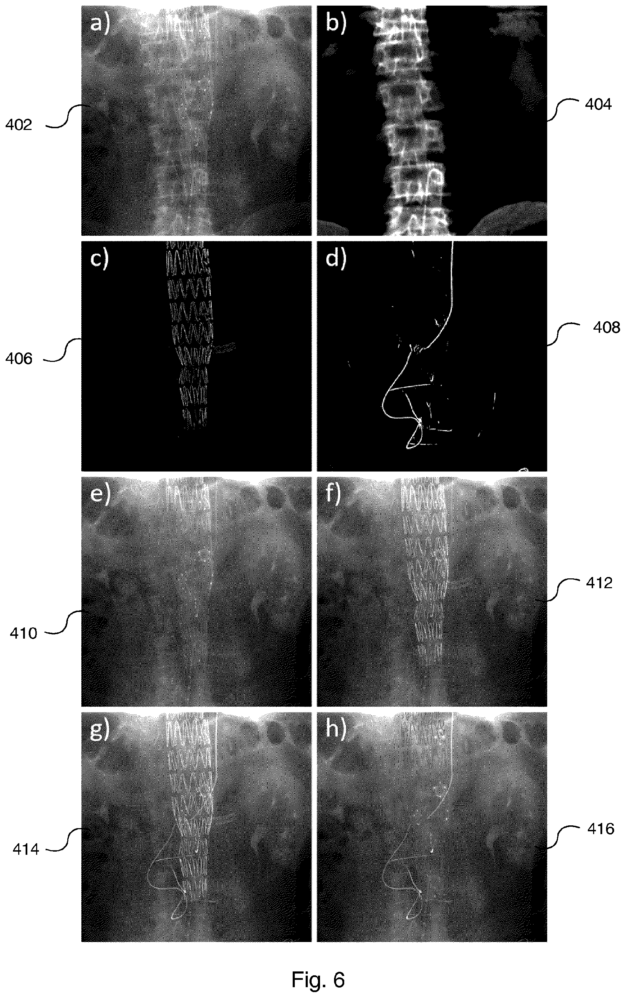

Figur 6 zeigt ein Eingangsbild 402, ein Knochen-Teilbild 404, ein Stent-Teilbild 406 und ein Führungsdraht-Teilbild 408. Im ersten Ausgabebild 410 wurde die Sichtbarkeit von Knochen verringert. Im zweiten Ausgabebild 412 wurde zusätzlich die Sichtbarkeit eines Stents angehoben. Im dritten Ausgabebild 414 wurde zusätzlich die Sichtbarkeit von Führungsdrähten angehoben. Im vierten Ausgabebild 416 wurde der Stent zudem farblich hervorgehoben, indem das Stent-Teilbild 406 vor dem Zusammenmischen eingefärbt wurde.

Das Zusammenmischen des Eingangsbildes 402 und der Teilbilder 404, 406 und 408 erfolgt mit dem Ziel, dass im resultierenden Ausgabebild 410, 412, 414, 416 die Sichtbarkeit von Strukturen bestimmter semantischer Klassen bezogen auf die Sichtbarkeit im Eingangsbild vergrößert oder verringert wird. Ob und in welchem Maße die Sichtbarkeit von Strukturen einer gegebenen semantischen Klasse vergrößert oder verringert wird, richtet sich idealerweise nach den aktuellen Bedürfnissen des Betrachters. Beispielsweise könnte dies in einem Organprogramm festgelegt werden. Beispielsweise ist es bei gefäßchirurgischen Eingriffen wahrscheinlich, dass die Sichtbarkeit von Knochen verringert und die Sichtbarkeit von Interventionsmaterial (beispielsweise Stents, Führungsdrähte etc.) erhöht werden soll, oder dass nur Kontrastmittel-haltige Blutgefäße und Interventionsmaterial angezeigt werden sollen.

In Figur 6 sind verschiedene Möglichkeiten des Zusammenmischens des Eingangsbildes 402, des Knochen-Teilbildes 404, des Stent-Teilbildes 406 und des Führungsdraht-Teilbildes 408 gezeigt. Eine weitere Möglichkeit festzulegen, ob und wie stark die Sichtbarkeit einer semantischen Klasse verändert werden soll, besteht darin, dem Betrachter ein Bedienelement beispielsweise auf einem berührungsempfindlichen Bildschirm zur Verfügung zu stellen. Richtung und Ausmaß der Betätigung des Bedienelements würden dann steuern, ob und wie stark die Sichtbarkeit verringert oder erhöht wird (erfindungsgemäßer semantischer Kontrastregler).

Weiterhin kann es vorteilhaft sein, die Mischung automatisch und in Echtzeit zu bestimmen, beispielsweise weil man dem Benutzer die Aufgabe, die Mischung einzustellen, nicht zumuten möchte und die evtl. im Organprogramm festgelegte Mischung nicht in jeder Situation zufriedenstellend ist. Dieses automatische Einstellen kann beispielsweise durch Optimieren einer Zielfunktion erfolgen, beispielsweise durch ein Gradienten-basiertes Optimierungsverfahren, ein Nelder-Mead-Optimierungsverfahren oder Verfahren aus dem Bereich der nicht-konvexen oder globalen Optimierung. Beispielsweise könnte eine solche Zielfunktion auf den Histogrammen der Bilder basieren und/oder den typischen Kontrast zwischen den Strukturen verschiedener semantischer Klassen und/oder Strukturen des Eingangsbilds messen. Auch ist es denkbar, die Zielfunktion nur auf bestimmten Regionen der Bilder zu berechnen, wobei die Regionen aus einem oder mehreren der Teilbilder berechnet werden (beispielsweise durch Schwellwertsetzung und morphologische Operationen). Weil das Optimieren für eine Echtzeit-Anwendung zu langsam sein kann, kann es vorteilhaft sein, andere, schnellere Algorithmen zur Bestimmung der Mischungsfaktoren zu verwenden. Beispielsweise könnte ein MLV, das darauf trainiert wurde die optimale Mischung zu schätzen, verwendet werden.

Praktisch kann das Zusammenmischen beispielsweise durch Linearkombination von Eingangsbild 102 und Teilbildern 104, 106, 108 erfolgen, wie in Figur 2 gezeigt ist.

Unter Linearkombination verstehen die Erfinder, dass jedes der vorgenannten Bilder mit einem i.A. reellwertigen Faktor (positiv, null oder negativ) multipliziert wird und die Bilder anschließend addiert werden.

Vor dem Zusammenmischen können die Bilder ein- oder umgefärbt werden. Beispielsweise könnten Stents rot erscheinen, während die restlichen Bildinhalte in Graustufen erscheinen (siehe 416 in Figur 6). Verschiedene Farbkanäle können beim Zusammenmischen verschieden behandelt werden. Vor dem Zusammenmischen können Eingangsbild und/oder die Teilbilder auch anderweitig verarbeitet werden. Beispielsweise könnten, weil die Berechnung der Teilbilder nicht perfekt funktioniert, Teile eines Führungsdrahts im Knochen-Teilbild enthalten sein (falsch-positive Resultate, s.o.). Würde man dann die Sichtbarkeit von Knochen verringern, so würde man gleichzeitig die Sichtbarkeit des Führungsdrahts verringern. Um dies zu verhindern, könnte es beispielsweise vorteilhaft sein, eventuell vorhandene Führungsdrähte aus dem Knochen-Teilbild zu entfernen, beispielsweise durch Anwenden eines Tiefpassfilters auf das Knochenbild.

Erfindungsgemäß wird eingestellt, ob und wie stark die Sichtbarkeit einer semantischen Klasse verändert werden soll. Eine Möglichkeit besteht darin, dem Betrachter ein Bedienelement beispielsweise auf einem berührungsempfindlichen Bildschirm zur Verfügung zu stellen. Richtung und Ausmaß der Betätigung des Bedienelements würden dann steuern, ob und wie stark die Sichtbarkeit verringert oder erhöht wird (erfindungsgemäßer semantischer Kontrastregler). Eine weitere Möglichkeit ist, die Sichtbarkeit einer semantischen Klasse in Abhängigkeit des medizinischen Eingriffs festzulegen.

## Patentansprüche

1. Verfahren zum Einstellen der Sichtbarkeit von Objekten in einem durch Strahlung erzeugten Projektionsbild eines anatomischen Bereichs:
- Laden von Bilddaten (102), die das durch Strahlung erzeugte Projektionsbild repräsentieren, in einen Speicher eines Computers;
- Berechnen eines ersten Teilbildes (104) aus den Bilddaten (102) unter Verwendung einer ersten semantischen Klasse durch eine Künstliche-Intelligenz-Einrichtung mittels eines Künstliche-Intelligenz-Algorithmus, wobei die erste semantische Klasse zumindest eine der folgenden semantischen Klassen umfasst: eine Repräsentation einer Abbildung einer anatomischen Struktur (101), eine Repräsentation einer Abbildung eines medizinischen Implantats (103), eine Repräsentation einer Abbildung eines medizinischen Werkzeugs (109) und eine Repräsentation einer Abbildung eines medizinischen Befestigungselements;
- Mischen des ersten Teilbildes (104) und der Bilddaten (102), um ein Ausgabebild (118) unter Einsatz einer ersten einstellbaren Funktion mit zumindest einem ersten Parameter zu erzeugen, wobei der zumindest eine erste Parameter durch zumindest eine Benutzereingabe und/oder dem Typ eines zu untersuchenden anatomischen Bereichs und/oder der ersten semantischen Klasse und/oder der Art des medizinischen Eingriffs ausgewählt wird und wobei die erste Funktion die Darstellung des ersten Teilbildes im Ausgabebild bestimmt; und
- Ausgeben des Ausgabebildes auf einer Anzeigevorrichtung (16);
- ferner aufweisend die folgenden Schritte zum Erzeugen von Trainingsdaten zum Trainieren des Künstliche-Intelligenz-Algorithmus der Künstliche-Intelligenz-Einrichtung:
- Erzeugen der Trainingsdaten zum Trainieren des Künstliche-Intelligenz-Algorithmus der Künstliche-Intelligenz-Einrichtung mittels mindestens einem Satz aus einem Eingangsbild (304, 320) und einem ersten Zielbild (310, 322);
- Erzeugen des Zwischenbildes (306) durch Ersetzen in einem Bereich zumindest eines Eingangsbildes (304, 320) oder eines Bereichs eines Bildmodells, aus dem das Eingangsbild erzeugt wird, von Bilddaten, die die erste semantischen Klasse darstellen, durch Bilddaten, die das umgebende Gewebe darstellen; und
- Erzeugen des ersten Zielbildes (310, 322) durch Subtraktion des Zwischenbildes (306) vom Eingangsbild (304, 320);
- wobei jedes Eingangsbild (304, 320) einem Projektionsbild des zu untersuchenden anatomischen Bereichs entspricht, das durch Strahlung erzeugt wird oder dessen Erzeugung durch Strahlung teilweise oder vollständig simuliert wird; und
- wobei zumindest ein erstes Zielbild (310, 322) Bildinformation der ersten semantischen Klasse aufweist;
ferner aufweisend den Schritt des Trainierens des Künstliche-Intelligenz-Algorithmus der Künstliche-Intelligenz-Einrichtung mittels der Trainingsdaten.

2. Verfahren nach Anspruch 1, wobei die Darstellung des ersten Teilbildes (104) im Ausgabebild (118) zumindest die Sichtbarkeit und/oder die Helligkeit und/oder den Kontrast und/oder die Farbe des ersten Teilbildes im Ausgabebild umfasst.

3. Verfahren nach Anspruch 1 oder 2, wobei der Schritt des Erzeugens der Trainingsdaten für den Künstliche-Intelligenz-Algorithmus der Künstliche-Intelligenz-Einrichtung auf einer Künstliche-Intelligenz-Trainingseinrichtung durchgeführt werden.

4. Verfahren nach einem der Ansprüche 1 bis 3, wobei jedes Eingangsbild auch Bildinformation der ersten semantischen Klasse aufweist.

5. Verfahren nach einem der Ansprüche 1 bis 4, ferner aufweisend die folgenden Schritte:
- Berechnen eines zweiten Teilbildes (106) aus den Bilddaten unter Verwendung einer zweiten semantischen Klasse durch die Künstliche-Intelligenz-Einrichtung mittels des Künstliche-Intelligenz-Algorithmus,
wobei die zweite semantische Klasse zumindest eine der folgenden semantischen Klassen umfasst: eine Repräsentation einer Abbildung einer anatomischen Struktur, eine Repräsentation einer Abbildung eines medizinischen Implantats, eine Repräsentation einer Abbildung eines medizinischen Werkzeugs und eine Repräsentation einer Abbildung eines medizinischen Befestigungselements;
- Mischen des ersten Teilbildes (104), des zweiten Teilbildes (106) und der Bilddaten (103), um das Ausgabebild unter Einsatz einer Funktion mit der ersten einstellbaren Funktion mit dem zumindest einen ersten Parameter und mit einer zweiten einstellbaren Funktion mit zumindest einem zweiten Parameter zu erzeugen und wobei der zumindest eine zweite Parameter durch eine Benutzereingabe und/oder dem Typ des zu untersuchenden anatomischen Bereichs und/oder der zweiten semantischen Klasse und/oder der Art des medizinischen Eingriffs ausgewählt wird und wobei die zweite Funktion die Darstellung des zweiten Teilbildes im Ausgabebild (118) bestimmt.

6. Verfahren nach Anspruch 5, ferner aufweisend die folgenden Schritte:
- Berechnen eines n-ten Teilbildes (108) aus den Bilddaten unter Verwendung einer n-ten semantischen Klasse durch die Künstliche-Intelligenz-Einrichtung_mittels des Künstliche-Intelligenz-Algorithmus, wobei die n-te semantische Klasse zumindest eine der folgenden semantischen Klassen umfasst: eine Repräsentation einer Abbildung einer anatomischen Struktur, eine Repräsentation einer Abbildung eines medizinischen Implantats, eine Repräsentation einer Abbildung eines medizinischen Werkzeugs und eine Repräsentation einer Abbildung eines medizinischen Befestigungselements;
- Mischen der Teilbilder 1 bis n (104, 106, 108) mit den Bilddaten (103), um das Ausgabebild (118) unter Einsatz einer Funktion mit n einstellbaren Funktion mit je zumindest einem Parameter zu erzeugen und wobei der jeweilige zumindest eine Parameter durch zumindest eine Benutzereingabe und/oder dem Typ des anatomischen Bereichs und/oder der n-ten semantischen Klasse und/oder der Art des medizinischen Eingriffs ausgewählt wird und wobei die erste bis n-te Funktion die Darstellung des ersten bis n-ten Teilbildes (104, 106, 108) im Ausgabebild (118) bestimmt.

7. Verfahren nach einem der Ansprüche 1 bis 6, wobei das Eingangsbild (102) aus einem dreidimensionalen Bildmodell eines Gewebes eines Patienten erzeugt wird.

8. Verfahren nach einem der Ansprüche 1 bis 7, wobei der Schritt des Berechnens des ersten Zielbildes (310, 322) folgende Schritte aufweist:
- Auswählen einer semantischen Klasse als ausgewählte Klasse, deren Bildinformation in dem ersten Zielbild erhalten sein soll;
- Laden eines ersten dreidimensionalen Bildmodells des Gewebes eines Patienten in den Speicher des Computers, wobei das erste dreidimensionale Bildmodell des Gewebes des Patienten dreidimensionale Bildinformation der ausgewählten semantischen Klasse aufweist;
- Erzeugen eines zweiten dreidimensionalen Bildmodells, das keine Bildinformation der ausgewählten semantischen Klasse aufweist, durch Ersetzen in einem Bereich des ersten dreidimensionalen Bildmodells des Gewebes des Patienten von Bildinformationen, die die ausgewählte semantischen Klasse darstellt, durch Bildinformationen, die das umgebende Gewebe darstellt;
- Erzeugen des Eingangsbildes (304) aus dem ersten dreidimensionalen Bildmodell des Gewebes des Patienten;
- Erzeugen mindestens eines Zwischenbildes (306) aus dem zweiten dreidimensionalen Bildmodell; und
- Erzeugen des mindestens einen ersten Zielbildes (310, 322) durch Subtrahieren des Zwischenbildes (306) vom Eingangsbild.

9. Verfahren nach Anspruch 8, wobei
- das dreidimensionale Bildmodell des Gewebes des Patienten mittels einer dreidimensionalen Röntgenbildgebung erzeugt wird; oder
- das dreidimensionale Bildmodell des Gewebes des Patienten mittels einer Simulation einer dreidimensionalen Röntgenbildgebung erzeugt wird.

10. Verfahren nach Anspruch 8 oder 9, wobei
- die dreidimensionale Bildinformation der ausgewählten semantischen Klasse mittels einer dreidimensionalen Röntgenbildgebung erzeugt wird; oder
- die dreidimensionale Bildinformation der ausgewählten semantischen Klasse mittels einer Simulation einer dreidimensionalen Röntgenbildgebung erzeugt wird.

11. Verfahren nach einem der Ansprüche 1 bis 10, ferner aufweisend die folgenden Schritte:
- Einstellen des ersten Parameters mittels einer ersten Bedieneinrichtung; und/oder
- Einstellen des zweiten Parameters mittels einer zweiten Bedieneinrichtung; und/oder
- Einstellen zumindest eines n-ten Parameters mittels zumindest einer n-ten Bedieneinrichtung.

12. Verfahren nach einem der Ansprüche 1 bis 11, wobei der erste Parameter und/oder der zweite Parameter und/oder der n-te Parameter ein Gewichtungsfaktor ist.

13. Verfahren nach einem der Ansprüche 5 bis 12, wobei das Ausgabebild wie folgt berechnet wird: 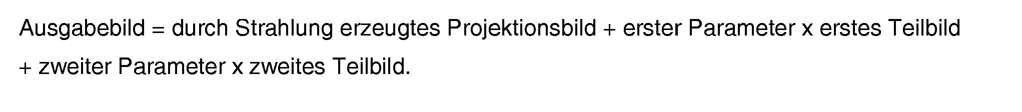 Ausgabebild = durch Strahlung erzeugtes Projektionsbild + erster Parameter x erstes Teilbild + zweiter Parameter x zweites Teilbild.

14. Verfahren nach einem der Ansprüche 5 bis 13, wobei der Satz ein zweites Zielbild (322) aufweist, das Bildinformation der zweiten semantischen Klasse aufweist.

15. Medizinische Bildgebungsvorrichtung, aufweisend:
- eine Strahlenquelle (13), die Strahlung emittiert, und einen Strahlungssensor (12), auf den die Strahlung auftrifft, nachdem sie ein anatomisches Gewebe passiert hat, wobei der Strahlungssensor (12) Strahlungsdaten ausgibt;
- eine Projektionsbilderzeugungseinrichtung, die aus Strahlungsdaten ein Projektionsbild erzeugt;
- einen Speicher (22), in den Bilddaten, die das durch Strahlung erzeugte Projektionsbild repräsentieren, geladen werden;
- eine Künstliche-Intelligenz-Einrichtung, die zum Berechnen eines ersten Teilbildes aus den Bilddaten unter Verwendung einer ersten semantischen Klasse mittels eines Künstliche-Intelligenz-Algorithmus ausgebildet ist, wobei die erste semantische Klasse zumindest eine der folgenden semantischen Klassen umfasst: eine Repräsentation einer Abbildung einer anatomischen Struktur, eine Repräsentation einer Abbildung eines medizinischen Implantats, eine Repräsentation einer Abbildung eines medizinischen Werkzeugs und eine Repräsentation einer Abbildung eines medizinischen Befestigungselements;
- eine Mischeinrichtung, die zum Mischen des ersten Teilbildes und der Bilddaten ausgebildet ist, um ein Ausgabebild unter Einsatz einer ersten einstellbaren Funktion mit zumindest einem ersten Parameter zu erzeugen, wobei der zumindest eine erste Parameter durch zumindest eine Benutzereingabe und/oder dem Typ eines zu untersuchenden anatomischen Bereichs und/oder der ersten semantischen Klasse und/oder der Art des medizinischen Eingriffs ausgewählt wird; und
- eine Anzeigevorrichtung (16), die zum Ausgeben des Ausgabebildes ausgebildet ist;
- wobei eine Künstliche-Intelligenz-Trainingseinrichtung dazu ausgebildet ist,
- Trainingsdaten zum Trainieren des Künstliche-Intelligenz-Algorithmus der Künstliche-Intelligenz-Einrichtung mittels mindestens einem Satz aus einem Eingangsbild (304, 320) und einem ersten Zielbild (310, 322) zu erzeugen; und
- ein Zwischenbild (306) durch Ersetzen in einem Bereich zumindest eines Eingangsbildes (304, 320) oder eines Bereichs eines Bildmodells zu erzeugen, aus dem das Eingangsbild erzeugt wird, von Bilddaten, die die erste semantischen Klasse darstellen, durch Bilddaten, die das umgebende Gewebe darstellen;
- das erste Zielbild (310, 322) durch Subtraktion des Zwischenbildes (306) vom Eingangsbild (304, 320) zu erzeugen; und
- den Künstlichen-Intelligenz-Algorithmus der Künstlichen-Intelligenz-Einrichtung mittels der Trainingsdaten zu trainieren;
- wobei jedes Eingangsbild (304, 320) einem Projektionsbild des zu untersuchenden anatomischen Bereichs entspricht, das durch Strahlung erzeugt wird oder dessen Erzeugung durch Strahlung teilweise oder vollständig simuliert wird; und
- wobei zumindest ein erstes Zielbild (310, 322) Bildinformation der ersten semantischen Klasse aufweist.

## Claims

1. A method for adjusting the visibility of objects in a radiation-generated projection image of an anatomical region:
- loading image data (102) representing the radiation-generated projection image into a memory of a computer;
- calculating a first subimage (104) from the image data (102) using a first semantic class via an artificial intelligence device by means of an artificial intelligence algorithm, wherein the first semantic class comprises at least one of the following semantic classes: a representation of an image of an anatomical structure (101), a representation of an image of a medical implant (103), a representation of an image of a medical instrument (109), and a representation of an image of a medical fastener;
- mixing the first partial image (104) and the image data (102), to generate an output image (118) using a first adjustable function with at least one first parameter, wherein the at least one first parameter is selected based on at least one user input and/or the type of anatomical region to be examined and/or the first semantic class and/or the type of medical procedure, and wherein the first function determines the representation of the first partial image in the output image; and
- outputting the output image to a display device (16);
- further comprising the following steps for generating training data to train the artificial intelligence algorithm of the artificial intelligence device:
- generating the training data for training the artificial intelligence algorithm of the artificial intelligence device using at least one set comprising an input image (304, 320) and a first target image (310, 322);
- generating the intermediate image (306) by replacing, within a region of at least one input image (304, 320) or a region of an image model from which the input image is generated, image data representing the first semantic class with image data representing the surrounding tissue; and
- generating the first target image (310, 322) by subtracting the intermediate image (306) from the input image (304, 320);
- wherein each input image (304, 320) corresponds to a projection image of the anatomical region under examination that is generated by radiation or whose generation by radiation is partially or fully simulated; and
- wherein at least a first target image (310, 322) comprises image information of the first semantic class;
further comprising the step of training the artificial intelligence algorithm of the artificial intelligence device using the training data.

2. The method according to claim 1, wherein the representation of the first sub-image (104) in the output image (118) comprises at least the visibility and/or the brightness and/or the contrast and/or the color of the first sub-image in the output image.

3. The method according to claim 1 or 2, wherein the step of generating the training data for the artificial intelligence algorithm of the artificial intelligence device is performed on an artificial intelligence training device.

4. The method according to any one of claims 1 through 3, wherein each input image also comprises image information of the first semantic class.

5. A method according to any one of claims 1 through 4, further comprising the following steps:
- calculating a second sub-image (106) from the image data using a second semantic class by the artificial intelligence device via the artificial intelligence algorithm,
- wherein the second semantic class comprises at least one of the following semantic classes: a representation of an image of an anatomical structure, a representation of an image of a medical implant, a representation of an image of a medical instrument, and a representation of an image of a medical fastener;
- mixing the first sub-image (104), the second sub-image (106), and the image data (103) to generate the output image using a function comprising the first adjustable function with the at least one first parameter and a second adjustable function with at least one second parameter, and wherein the at least one second parameter is selected based on user input and/ or the type of anatomical region to be examined and/or the second semantic class and/or the type of medical procedure, and wherein the second function determines the representation of the second partial image in the output image (118).

6. The method according to claim 5, further comprising the following steps:
- Calculating an nth subimage (108) from the image data using an nth semantic class by the artificial intelligence device by means of the artificial intelligence algorithm, wherein the nth semantic class comprises at least one of the following semantic classes: a representation of an image of an anatomical structure, a representation of an image of a medical implant, a representation of an image of a medical instrument, and a representation of an image of a medical fastener;
- mixing the partial images 1 through n (104, 106, 108) with the image data (103) to generate the output image (118) using a function comprising n adjustable functions, each with at least one parameter, wherein the respective at least one parameter is selected based on at least one user input and/or the type of anatomical region and/or the nth semantic class and/or the type of medical procedure, and wherein the first through nth functions determine the representation of the first through nth partial images (104, 106, 108) in the output image (118).

7. A method according to any one of claims 1 through 6, wherein the input image (102) is generated from a three-dimensional image model of a tissue of a patient.

8. A method according to any one of claims 1 through 7, wherein the step of calculating the first target image (310, 322) comprises the following steps:
- selecting a semantic class as the selected class, the image information of which is to be retained in the first target image;
- loading a first three-dimensional image model of the tissue of a patient into the memory of a computer, wherein the first three-dimensional image model of the tissue of the patient comprises three-dimensional image information of the selected semantic class;
- generating a second three-dimensional image model that does not comprise image information of the selected semantic class by replacing, within a region of the first three-dimensional image model of the tissue of the patient, image information representing the selected semantic class with image information representing the surrounding tissue;
- generating the input image (304) from the first three-dimensional image model of the tissue of the patient;
- generating at least one intermediate image (306) from the second three-dimensional image model; and
- generating the at least one first target image (310, 322) by subtracting the intermediate image (306) from the input image.

9. The method according to claim 8, wherein
- the three-dimensional image model of the tissue of the patient is generated by means of three-dimensional X-ray imaging; or
- the three-dimensional image model of the tissue of the patient is generated by means of a simulation of three-dimensional X-ray imaging.

10. The method according to claim 8 or 9, wherein
- the three-dimensional image information of the selected semantic class is generated by means of three-dimensional X-ray imaging; or
- the three-dimensional image information of the selected semantic class is generated by means of a simulation of three-dimensional X-ray imaging.

11. A method according to any one of claims 1 through 10, further comprising the following steps:
- setting the first parameter using a first control device; and/or
- setting the second parameter using a second control device; and/or
- setting at least an nth parameter using at least an nth control device.

12. A method according to any one of claims 1 through 11, wherein the first parameter and/or the second parameter and/or the nth parameter is a weighting factor.

13. A method according to any one of claims 5 through 12, wherein the output image is calculated as follows: 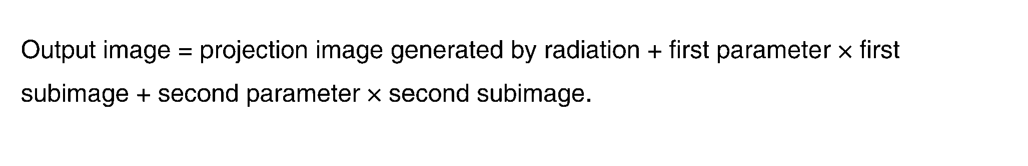 Output image = projection image generated by radiation + first parameter × first subimage + second parameter × second subimage.

14. A method according to any one of claims 5 through 13, wherein the set comprises a second target image (322) that comprises image information of the second semantic class.

15. A medical imaging device that comprises:
- a radiation source (13) that emits radiation, and a radiation sensor (12) onto which the radiation strikes after passing through anatomical tissue, wherein the radiation sensor (12) outputs radiation data;
- a projection image generation device that generates a projection image from the radiation data;
- a memory (22) into which image data representing the projection image generated by radiation is loaded;
- an artificial intelligence device adapted to calculate a first sub-image from the image data using a first semantic class by means of an artificial intelligence algorithm, wherein the first semantic class comprises at least one of the following semantic classes: a representation of an image of an anatomical structure, a representation of an image of a medical implant, a representation of an image of a medical instrument, and a representation of an image of a medical fastener;
- a mixing device adapted to mix the first partial image and the image data to generate an output image using a first adjustable function with at least one first parameter, wherein the at least one first parameter is selected based on at least one user input and/or the type of anatomical region to be examined and/or the first semantic class and/or the type of medical procedure; and
- a display device (16) adapted to output the output image;
- wherein an artificial intelligence training device is adapted to
- generate training data for training the artificial intelligence algorithm of the artificial intelligence device using at least one set comprising an input image (304, 320) and a first target image (310, 322); and
- generating an intermediate image (306) by replacing, within a region of at least one input image (304, 320) or a region of an image model from which the input image is generated, image data representing the first semantic class with image data representing the surrounding tissue;
- generating the first target image (310, 322) by subtracting the intermediate image (306) from the input image (304, 320); and
- training the artificial intelligence algorithm of the artificial intelligence device using the training data;
- wherein each input image (304, 320) corresponds to a projection image of the anatomical region under examination that is generated by radiation or whose generation by radiation is partially or completely simulated; and
- wherein at least a first target image (310, 322) comprises image information of the first semantic class.

## Revendications

1. Procédé pour régler la visibilité d'objets dans une image de projection générée par rayonnement d'une région anatomique, le procédé comprenant :
- le chargement de données d'image (102) qui représentent l'image de projection générée par rayonnement à l'intérieur d'une mémoire d'un ordinateur ;
- le calcul d'une première sous-image (104) à partir des données d'image (102) en utilisant une première classe sémantique par l'intermédiaire d'un dispositif d'intelligence artificielle qui fonctionne sur la base d'un algorithme d'intelligence artificielle, dans lequel la première classe sémantique comprend au moins l'une des classes sémantiques suivantes : une représentation d'une image d'une structure anatomique (101), une représentation d'une image d'un implant médical (103), une représentation d'une image d'un instrument médical (109) et une représentation d'une image d'une pièce de fixation médicale ;
- le mélange de la première sous-image ou image partielle (104) et des données d'image (102) afin de générer une image de sortie (118) en utilisant une première fonction réglable qui est pourvue d'au moins un premier paramètre, dans lequel l'au moins un premier paramètre est sélectionné sur la base d'au moins une entrée d'utilisateur et/ou du type de région anatomique à examiner et/ou de la première classe sémantique et/ou du type de procédure médicale, et dans lequel la première fonction détermine la représentation de la première sous-image ou image partielle dans l'image de sortie ; et
- l'émission en sortie de l'image de sortie sur un dispositif d'affichage (16) ; comprenant en outre les étapes suivantes pour générer des données d'entraînement afin d'entraîner l'algorithme d'intelligence artificielle du dispositif d'intelligence artificielle :
- la génération des données d'entraînement pour entraîner l'algorithme d'intelligence artificielle du dispositif d'intelligence artificielle en utilisant au moins un ensemble qui comprend une image d'entrée (304, 320) et une première image cible (310, 322) ;
- la génération d'une image intermédiaire (306) en remplaçant, à l'intérieur d'une région d'au moins une image d'entrée (304, 320) ou à l'intérieur d'une région d'un modèle d'image à partir duquel l'image d'entrée est générée, des données d'image qui représentent la première classe sémantique par des données d'image qui représentent le tissu avoisinant ; et
- la génération de la première image cible (310, 322) en soustrayant l'image intermédiaire (306) de l'image d'entrée (304, 320) ;
dans lequel chaque image d'entrée (304, 320) correspond à une image de projection de la région anatomique en cours d'examen qui est générée par rayonnement ou dont la génération par rayonnement est simulée en partie ou en totalité ; et
dans lequel au moins une première image cible (310, 322) comprend une information d'image de la première classe sémantique ;
comprenant en outre l'étape d'entraînement de l'algorithme d'intelligence artificielle du dispositif d'intelligence artificielle en utilisant les données d'entraînement.

2. Procédé selon la revendication 1, dans lequel la représentation de la première sous-image (104) dans l'image de sortie (118) comprend au moins la visibilité et/ou la luminosité et/ou le contraste et/ou la couleur de la première sous-image dans l'image de sortie.

3. Procédé selon la revendication 1 ou 2, dans lequel l'étape de génération des données d'entraînement pour l'algorithme d'intelligence artificielle du dispositif d'intelligence artificielle est réalisée sur un dispositif d'entraînement d'intelligence artificielle.

4. Procédé selon l'une quelconque des revendications 1 à 3, dans lequel chaque image d'entrée comprend également une information d'image de la première classe sémantique.

5. Procédé selon l'une quelconque des revendications 1 à 4, comprenant en outre les étapes suivantes :
- le calcul d'une seconde sous-image (106) à partir des données d'image en utilisant une seconde classe sémantique par l'intermédiaire du dispositif d'intelligence artificielle qui fonctionne sur la base de l'algorithme d'intelligence artificielle,
dans lequel la seconde classe sémantique comprend au moins l'une des classes sémantiques suivantes : une représentation d'une image d'une structure anatomique, une représentation d'une image d'un implant médical, une représentation d'une image d'un instrument médical et une représentation d'une image d'une pièce de fixation médicale ; et
- le mélange de la première sous-image (104), de la seconde sous-image (106) et des données d'image (103) afin de générer l'image de sortie en utilisant une fonction qui comprend la première fonction réglable qui est pourvue de l'au moins un premier paramètre et une seconde fonction réglable qui est pourvue d'au moins un second paramètre, et dans lequel l'au moins un second paramètre est sélectionné sur la base d'une entrée d'utilisateur et/ou du type de région anatomique à examiner et/ou de la seconde classe sémantique et/ou du type de procédure médicale, et dans lequel la seconde fonction détermine la représentation de la seconde sous-image ou image partielle dans l'image de sortie (118).

6. Procédé selon la revendication 5, comprenant en outre les étapes suivantes :
- le calcul d'une n-ième sous-image (108) à partir des données d'image en utilisant une n-ième classe sémantique par l'intermédiaire du dispositif d'intelligence artificielle qui fonctionne sur la base de l'algorithme d'intelligence artificielle, dans lequel la n-ième classe sémantique comprend au moins l'une des classes sémantiques suivantes : une représentation d'une image d'une structure anatomique, une représentation d'une image d'un implant médical, une représentation d'une image d'un instrument médical et une représentation d'une image d'une pièce de fixation médicale ; et
- le mélange des sous-images ou images partielles 1 à n (104, 106, 108) avec les données d'image (103) afin de générer l'image de sortie (118) en utilisant une fonction qui comprend n fonctions réglables dont chacune est pourvue d'au moins un paramètre, dans lequel l'au moins un paramètre respectif est sélectionné sur la base d'au moins une entrée utilisateur et/ou du type de région anatomique et/ou de la n-ième classe sémantique et/ou du type de procédure médicale, et dans lequel les première à n-ième fonctions déterminent la représentation des première à n-ième sous-images ou images partielles (104, 106, 108) dans l'image de sortie (118).

7. Procédé selon l'une quelconque des revendications 1 à 6, dans lequel l'image d'entrée (102) est générée à partir d'un modèle d'image tridimensionnelle d'un tissu d'un patient.

8. Procédé selon l'une quelconque des revendications 1 à 7, dans lequel l'étape de calcul de la première image cible (310, 322) comprend les étapes suivantes :
- la sélection d'une classe sémantique en tant que classe sélectionnée dont l'information d'image doit être retenue dans la première image cible ;
- le chargement d'un premier modèle d'image tridimensionnelle du tissu d'un patient à l'intérieur de la mémoire d'un ordinateur, dans lequel le premier modèle d'image tridimensionnelle du tissu du patient comprend une information d'image tridimensionnelle de la classe sémantique sélectionnée ;
- la génération d'un second modèle d'image tridimensionnelle qui ne comprend pas d'information d'image de la classe sémantique sélectionnée en remplaçant, à l'intérieur d'une région du premier modèle d'image tridimensionnelle du tissu du patient, une information d'image qui représente la classe sémantique sélectionnée par une information d'image qui représente le tissu avoisinant ;
- la génération de l'image d'entrée (304) à partir du premier modèle d'image tridimensionnelle du tissu du patient ;
- la génération d'au moins une image intermédiaire (306) à partir du second modèle d'image tridimensionnelle ; et
- la génération de l'au moins une première image cible (310, 322) en soustrayant l'image intermédiaire (306) de l'image d'entrée.

9. Procédé selon la revendication 8, dans lequel :
- le modèle d'image tridimensionnelle du tissu du patient est généré au moyen d'une imagerie rayons X tridimensionnelle ; ou
- le modèle d'image tridimensionnelle du tissu du patient est généré au moyen d'une simulation d'imagerie rayons X tridimensionnelle.

10. Procédé selon la revendication 8 ou 9, dans lequel :
- l'information d'image tridimensionnelle de la classe sémantique sélectionnée est générée au moyen d'une imagerie rayons X tridimensionnelle ; ou
- l'information d'image tridimensionnelle de la classe sémantique sélectionnée est générée au moyen d'une simulation d'imagerie rayons X tridimensionnelle.

11. Procédé selon l'une quelconque des revendications 1 à 10, comprenant en outre les étapes suivantes :
- la définition du premier paramètre en utilisant un premier dispositif de commande ; et/ou
- la définition du second paramètre en utilisant un second dispositif de commande ; et/ou
- la définition d'au moins un n-ième paramètre en utilisant au moins un n-ième dispositif de commande.

12. Procédé selon l'une quelconque des revendications 1 à 11, dans lequel le premier paramètre et/ou le second paramètre et/ou le n-ième paramètre sont ou est un facteur de pondération.

13. Procédé selon l'une quelconque des revendications 5 à 12, dans lequel l'image de sortie est calculée comme suit : 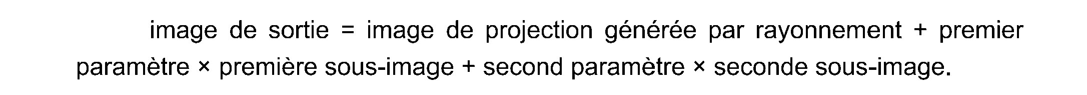 image de sortie = image de projection générée par rayonnement + premier paramètre × première sous-image + second paramètre × seconde sous-image.

14. Procédé selon l'une quelconque des revendications 5 à 13, dans lequel l'ensemble comprend une seconde image cible (322) qui comprend une information d'image de la seconde classe sémantique.

15. Dispositif d'imagerie médicale, lequel comprend :
- une source de rayonnement (13) qui émet un rayonnement et un capteur de rayonnement (12) sur lequel le rayonnement arrive en incidence après qu'il est passé au travers d'un tissu anatomique, dans lequel le capteur de rayonnement, (12) émet en sortie des données de rayonnement ;
- un dispositif de génération d'image de projection qui génère une image de projection à partir des données de rayonnement ;
- une mémoire (22) à l'intérieur de laquelle des données d'image qui représentent l'image de projection qui a été générée par rayonnement sont chargées ;
- un dispositif d'intelligence artificielle qui est adapté pour calculer une première sous-image à partir des données d'image en utilisant une première classe sémantique par l'intermédiaire d'un algorithme d'intelligence artificielle, dans lequel la première classe sémantique comprend au moins l'une des classes sémantiques suivantes : une représentation d'une image d'une structure anatomique, une représentation d'une image d'un implant médical, une représentation d'une image d'un instrument médical et une représentation d'une image d'une pièce de fixation médicale ;
- un dispositif de mélange qui est adapté pour mélanger la première sous-image ou image partielle et les données d'image afin de générer une image de sortie en utilisant une première fonction réglable qui est pourvue d'au moins un premier paramètre, dans lequel l'au moins un premier paramètre est sélectionné sur la base d'au moins une entrée d'utilisateur et/ou du type de région anatomique à examiner et/ou de la première classe sémantique et/ou du type de procédure médicale ; et
- un dispositif d'affichage (16) qui est adapté pour émettre en sortie l'image de sortie ;
dans lequel un dispositif d'entraînement d'intelligence artificielle est adapté pour :
- générer des données d'entraînement pour entraîner l'algorithme d'intelligence artificielle du dispositif d'intelligence artificielle en utilisant au moins un ensemble qui comprend une image d'entrée (304, 320) et une première image cible (310, 322) ; et
- générer une image intermédiaire (306) en remplaçant, à l'intérieur d'une région d'au moins une image d'entrée (304, 320) ou à l'intérieur d'une région d'un modèle d'image à partir duquel l'image d'entrée est générée, des données d'image qui représentent la première classe sémantique par des données d'image qui représentent le tissu avoisinant ;
- générer la première image cible (310, 322) en soustrayant l'image intermédiaire (306) de l'image d'entrée (304, 320) ; et
- entraîner l'algorithme d'intelligence artificielle du dispositif d'intelligence artificielle en utilisant les données d'entraînement ;
dans lequel chaque image d'entrée (304, 320) correspond à une image de projection de la région anatomique en cours d'examen qui est générée par rayonnement ou dont la génération par rayonnement est simulée en partie ou en totalité ; et
dans lequel au moins une première image cible (310, 322) comprend une information d'image de la première classe sémantique.
